# EUROPEAN PATENT SPECIFICATION

(11) **EP 4 478 992 B1**
(45) Date of publication and mention of the grant of the patent: **22.04.2026**
(21) Application number: 23718388.4
(22) Date of filing: 10.02.2023
(51) Int. Cl.: A61F 2/24, A61B 17/122

(54) **HEART VALVE REPAIR DEVICES AND DELIVERY DEVICES THEREFOR**
HERZKLAPPENREPARATURVORRICHTUNGEN UND FREISETZUNGSVORRICHTUNGEN DAFÜR
DISPOSITIFS DE RÉPARATION DE VALVULE CARDIAQUE ET DISPOSITIFS DE DISTRIBUTION ASSOCIÉS

(30) Priority: 15.02.2022 US 202263310553 P
(43) Date of publication of application: 25.12.2024
(73) Proprietor: Edwards Lifesciences Corporation, Irvine, CA 92614 (US)
(72) Inventor: CHU, Waina Michelle, Irvine, CA 92614 (US); PHAN, Jian Lin, Irvine, CA 92614 (US); OBERWISE, Eric Michael, Irvine, CA 92614 (US); DELGADO, Sergio, Irvine, CA 92614 (US); FRESCHAUF, Lauren R., Irvine, CA 92614 (US); CHEN, Wen Yan, Irvine, CA 92614 (US)
(74) Representative: Eisenführ Speiser
(86) International application number: PCT/US2023/012804
(87) International publication number: WO 2023/158593

(56) References cited:
- WO-A2-2018/195215
- US-A1- 2020 345 487
- US-A1- 2021 145 584

## Description

### BACKGROUND

The native heart valves (i.e., the aortic, pulmonary, tricuspid, and mitral valves) serve critical functions in assuring the forward flow of an adequate supply of blood through the cardiovascular system. These heart valves may be damaged, and thus rendered less effective, for example, by congenital malformations, inflammatory processes, infectious conditions, disease, etc. Such damage to the valves may result in serious cardiovascular compromise or death. Damaged valves may be surgically repaired or replaced during open heart surgery. However, open heart surgeries are highly invasive, and complications may occur. Transvascular techniques can be used to introduce and implant devices to treat a heart in a manner that is much less invasive than open heart surgery. As one example, a transvascular technique useable for accessing the native mitral and aortic valves is the trans-septal technique. The trans-septal technique comprises advancing a catheter into the right atrium (e.g., inserting a catheter into the right femoral vein, up the inferior vena cava and into the right atrium). The septum is then punctured, and the catheter passed into the left atrium. A similar transvascular technique can be used to implant a device within the tricuspid valve that begins similarly to the trans-septal technique but stops short of puncturing the septum and instead turns the delivery catheter toward the tricuspid valve in the right atrium.

A healthy heart has a generally conical shape that tapers to a lower apex. The heart is four-chambered and comprises the left atrium, right atrium, left ventricle, and right ventricle. The left and right sides of the heart are separated by a wall generally referred to as the septum. The native mitral valve of the human heart connects the left atrium to the left ventricle. The mitral valve has a very different anatomy than other native heart valves. The mitral valve includes an annulus portion, which is an annular portion of the native valve tissue surrounding the mitral valve orifice, and a pair of cusps, or leaflets, extending downward from the annulus into the left ventricle. The mitral valve annulus may form a "D"-shaped, oval, or otherwise out-of-round cross-sectional shape having major and minor axes. The anterior leaflet may be larger than the posterior leaflet, forming a generally "C"-shaped boundary between the abutting sides of the leaflets when they are closed together.

When operating properly, the anterior leaflet and the posterior leaflet function together as a one-way valve to allow blood to flow only from the left atrium to the left ventricle. The left atrium receives oxygenated blood from the pulmonary veins. When the muscles of the left atrium contract and the left ventricle dilates (also referred to as "ventricular diastole" or "diastole"), the oxygenated blood that is collected in the left atrium flows into the left ventricle. When the muscles of the left atrium relax and the muscles of the left ventricle contract (also referred to as "ventricular systole" or "systole"), the increased blood pressure in the left ventricle urges the sides of the two leaflets together, thereby closing the one-way mitral valve so that blood cannot flow back to the left atrium and is instead expelled out of the left ventricle through the aortic valve. To prevent the two leaflets from prolapsing under pressure and folding back through the mitral annulus toward the left atrium, a plurality of fibrous cords called chordae tendineae tether the leaflets to papillary muscles in the left ventricle.

Valvular regurgitation involves the valve improperly allowing some blood to flow in the wrong direction through the valve. For example, mitral regurgitation occurs when the native mitral valve fails to close properly and blood flows into the left atrium from the left ventricle during the systolic phase of heart contraction. Mitral regurgitation is one of the most common forms of valvular heart disease. Mitral regurgitation may have many different causes, such as leaflet prolapse, dysfunctional papillary muscles, stretching of the mitral valve annulus resulting from dilation of the left ventricle, more than one of these, etc. Mitral regurgitation at a central portion of the leaflets can be referred to as central jet mitral regurgitation and mitral regurgitation nearer to one commissure (i.e., location where the leaflets meet) of the leaflets can be referred to as eccentric jet mitral regurgitation. Central jet regurgitation occurs when the edges of the leaflets do not meet in the middle and thus the valve does not close, and regurgitation is present. Tricuspid regurgitation may be similar, but on the right side of the heart.

WO 2018/195215 A2 describes a prosthetic device which has a coaption element and at least one anchor. The coaption element is configured to be positioned within the native heart valve orifice to help fill a space where the native valve is regurgitant and form a more effective seal. The coaption element has a structure that is impervious to blood and that allows the native leaflets to close around the coaption element during ventricular systole to block blood from flowing from the left or right ventricle back into the left or right atrium, respectively. The coaption element can be connected to leaflets of the native valve by the anchor.

US 2020/0345487 A1 describes a method for use in the transvascular delivery and deployment of a prosthetic mitral valve. The method includes inverting an outer frame of a prosthetic mitral valve when the valve is in a biased expanded configuration. After inverting the outer frame, the prosthetic mitral valve is inserted into a lumen of a delivery sheath such that the mitral valve is moved to a collapsed configuration. The distal end portion of the delivery sheath is inserted into a left atrium of a heart. The prosthetic mitral valve is moved distally out of the delivery sheath such that the inverted outer frame reverts and the prosthetic mitral valve assumes its biased expanded configuration. In some embodiments, actuation wires are used to assist in the reversion of the outer frame. The prosthetic mitral valve is then positioned within a mitral annulus of the heart.

US 2021/0145584 A1 describes an apparatus comprising a tissue anchor for use with an anchor driver. The anchor comprises a head, and a tissue-engaging element coupled to a proximal end of the head. The tissue engaging element defines a central longitudinal axis of the anchor, and has a sharpened distal tip, configured to be driven into tissue of a subject. The head comprises a driver interface, configured to be reversibly engaged by the anchor driver, and an eyelet, disposed laterally from the central longitudinal axis, defining an aperture on an aperture plane, the aperture having a length along a long axis of the aperture and a width along a short axis of the aperture, the long axis and the short axis disposed on the aperture plane. The eyelet is mounted such that the aperture plane is slanted at a fixed angle with respect to the central longitudinal axis.

### SUMMARY

According to the present invention, an implantable device for repairing a native valve of a heart according to claim 1 is provided. Embodiments of the implantable device are defined by the dependent claims, the following description and the drawings.

This summary is meant to provide some examples and is not intended to be limiting of the scope of the invention in any way. For example, any feature included in an example of this summary is not required by the claims, unless the claims explicitly recite the feature. Also, the features, components, steps, concepts, etc. described in examples in this summary and elsewhere in this disclosure can be combined in a variety of ways. Various features and steps as described elsewhere in this disclosure may be included in the examples summarized here.

In some implementations according to the present invention, there is provided a device or implant (e.g., implantable device, etc.) that is configured to be positioned within a native heart valve to allow the native heart valve to form a more effective seal.

In some implementations, an implantable device or implant includes an anchor portion. Each anchor includes a plurality of paddles that are each moveable between an open position and a closed position.

In some implementations, a plurality of sutures is employed to secure outer frame portions of the paddles to a connector. Each of the sutures are passed through one or more eyelets of the outer frame portions and the connector. One or more sutures are optionally passed through a connecting anchor of the connector, which provides additional strength and stability to the interface.

According to the invention, a device for repairing a native valve of a heart includes an anchor portion, a connector, and a plurality of sutures. . The anchor portion is configured to connect to leaflets of the native valve. The anchor portion includes a pair of frame portions that are movable between an expanded position and a narrowed position. A frame width in the narrowed position is less than the frame width when in the expanded position.

Each of the pair of frame portions have an eyelet. The connector has an eyelet and a connecting anchor (e.g., tapered slot, locking aperture, etc.) proximate a first end of the connector.

The eyelet of the connector is positioned between the connecting anchor and the first end of the connector. The connector is at least partially positioned between the pair of frame portions such that the eyelets of the frame portions and the eyelet of the connector are aligned.

The plurality of sutures pass through the eyelet of the connector and the eyelet of each of the pair of frame portions and are configured to connect the pair of frame portions to the connector.

In some implementations, at least one of the plurality of sutures passes through the connecting anchor.

In some implementations, the plurality of sutures comprises at least two sutures passing through the connecting anchor.

In some implementations, the plurality of sutures pass through the eyelet of the connector and the eyelet of each of the pair of frame portions more than once.

In some implementations, one or more of the plurality of sutures is secured to itself.

In some implementations, the plurality of sutures comprises a first suture, a second suture, a third suture, and a fourth suture. In some implementations, the first suture and the second suture pass through the eyelet of the connector and the eyelet of each of the pair of frame portions and do not pass through the connecting anchor. In some implementations, the third suture and the fourth suture pass through the eyelet of the connector, the eyelet of each of the pair of frame portions, and the connecting anchor.

In some implementations, a sleeve is disposed about each of the frame portions.

In some implementations, each of the first and second sutures passes through the eyelet of the connector and the eyelet of each of the pair of frame portions, through the sleeve of the first frame portion and back through the eyelet of the connector and the eyelet of each of the pair of frame portions.

In some implementations, the pair of frame portions coaxially rotate relative to the connector while the frame portions move between the expanded position and the narrowed position.

In some implementations, the eyelets of the pair of frame portions and the eyelet of the connector are aligned along a common central axis while the frame portions move between the expanded position and the narrowed position.

The device can be used in method(s) of repairing a native valve of a heart. Such method(s) can be performed on a living animal or on a simulation, such as on a cadaver, cadaver heart, anthropomorphic ghost, simulator (e.g., with body parts, heart, tissue, etc. being simulated).

In some implementations, a device for repairing a native valve of a heart includes an anchor portion, a connector, a first plurality of sutures, and a second plurality of sutures. The anchor portion can be configured to connect to leaflets of the native valve. In some implementations, the anchor portion includes a pair of frame portions that are movable between an expanded position and a narrowed position.

In some implementations, the frame portions have a frame width that is less than the width of the frame portions when in the expanded position.

In some implementations, each of the pair of frame portions have an eyelet. In some implementations, the connector has an eyelet and a connecting anchor proximate a first end of the connector. In some implementations, the eyelet of the connector is positioned between the connecting anchor and the first end of the connector.

In some implementations, the connector is at least partially positioned between the pair of frame portions such that the eyelets of the frame portions and the eyelet of the connector are aligned.

In some implementations, the first plurality of sutures pass through the eyelet of the connector and the eyelet of each of the pair of frame portions. In some implementations, the second plurality of sutures pass through the eyelet of the connector, the eyelet of each of the pair of frame portions, and the connecting anchor.

In some implementations, the first plurality of sutures do not pass through the connecting anchor.

In some implementations, the first plurality of sutures and the second plurality of sutures are configured to attach the connector between the frame portions such that the pair of frame portions coaxially rotate relative to the connector while the frame portions move between the expanded position and the narrowed position.

In some implementations, the first plurality of sutures pass through the eyelet of the connector and the eyelet of each of the pair of frame portions more than once.

In some implementations, a sleeve is disposed about each of the frame portions.

In some implementations, at least some of the first plurality of sutures, the second plurality of sutures, or the first and second plurality of sutures pass through the sleeve of at least one of the frame portions.

In some implementations each suture of the first plurality of sutures is secured to itself.

The device can be used in method(s) of repairing a native valve of a heart. Such method(s) can be performed on a living animal or on a simulation, such as on a cadaver, cadaver heart, anthropomorphic ghost, simulator (e.g., with body parts, heart, tissue, etc. being simulated).

In some implementations, an implantable device includes an actuation cap having a flexible portion or a biasing element positioned between a rounded portion of the actuation cap and a retaining portion of the implantable device. In some implementations, the flexible portion or the biasing element flex or compress as a result of forces between the connection portion and the actuation cap, thereby reducing strain on the connector during delivery of the implantable device.

In some implementations, an implantable device includes a cap and an anchor portion. In some implementations, the cap has a hole traversing the cap from a proximal end to a distal end. In some implementations, the anchor portion is configured to connect to leaflets of a native valve. In some implementations, the anchor portion optionally includes a paddle frame, a connector, and a biasing element. In some implementations, the connector is coupled to the paddle frame. In some implementations, the connector has an inner end that extends through the hole in the cap.

In some implementations, pulling the inner end into the cap moves the paddle frame from an expanded position having an expanded width to a narrowed position having a narrowed width. In some implementations, the expanded width is greater than the narrowed width.

In some implementations, a biasing element is positioned between the cap and the anchor portion. In some implementations, the biasing element is configured to reduce a force between the cap and the paddle frame.

In some implementations, the anchor portion is configured to move between an open position and a closed position by movement of the cap.

In some implementations, the cap comprises a rounded portion extending between a coaptation element and the paddle frame and an attachment portion extending into the coaptation element. The attachment portion is configured to couple the cap to the coaptation element.

In some implementations, the biasing element is positioned between the rounded portion of the cap and the coaptation element.

In some implementations, the biasing element comprises a coil spring and/or a wave spring.

In some implementations, the biasing element is configured to reduce a force between the cap and the connector when the inner end of the connector moves into the cap to move the frame portions from the expanded position to the narrowed position.

The device can be used in method(s) of repairing a native valve of a heart. Such method(s) can be performed on a living animal or on a simulation, such as on a cadaver, cadaver heart, anthropomorphic ghost, simulator (e.g., with body parts, heart, tissue, etc. being simulated).

In some implementations, an implantable device includes a cap and an anchor portion. the cap has a hole traversing the cap from a proximal end to a distal end. In some implementations, the cap has one or more flexible portions at the distal end that extend away from the hole. In some implementations, the anchor portion is configured to connect to leaflets of a native valve. In some implementations, the anchor portion includes a paddle frame and a connector.

In some implementations, the connector is coupled to the paddle frame. In some implementations, the connector has an inner end that extends through the hole in the cap.

In some implementations, pulling the inner end into the cap moves the paddle frame from an expanded position having an expanded width to a narrowed position having a narrowed width.

In some implementations, the expanded width is greater than the narrowed width. The anchor portion is configured to move between an open position and a closed position by movement of the cap.

In some implementations, the one or more flexible portions are positioned between the frame portions and a coaptation element.

In some implementations, flexure of the one or more flexible portions reduces a force between the cap and the connector.

In some implementations, the flexible portions flex toward a coaptation element as the inner end is pulled into the cap to move the paddle frame from the expanded position to the narrowed position.

The device can be used in method(s) of repairing a native valve of a heart. Such method(s) can be performed on a living animal or on a simulation, such as on a cadaver, cadaver heart, anthropomorphic ghost, simulator (e.g., with body parts, heart, tissue, etc. being simulated).

Any of the above systems, assemblies, devices, apparatuses, components, etc. can be sterilized (e.g., with heat, radiation, ethylene oxide, hydrogen peroxide, etc.) to ensure they are safe for use with patients, and the above methods can comprise (or additional methods comprise or consist of) sterilization of one or more systems, devices, apparatuses, components, etc. herein (e.g., with heat, radiation, ethylene oxide, hydrogen peroxide, etc.).

A further understanding of the nature and advantages of the present invention are set forth in the following description and claims, particularly when considered in conjunction with the accompanying drawings in which like parts bear like reference numerals.

### BRIEF DESCRIPTION OF THE DRAWINGS

To further clarify various aspects of implementations of the present disclosure, a more particular description of the certain examples and implementations will be made by reference to various aspects of the appended drawings. These drawings depict only example implementations of the present disclosure and are therefore not to be considered limiting of the scope of the disclosure. Moreover, while the FIGS. can be drawn to scale for some examples, the FIGS. are not necessarily drawn to scale for all examples. Examples and other features and advantages of the present disclosure will be described and explained with additional specificity and detail through the use of the accompanying drawings in which:
FIG. 1 illustrates a cutaway view of the human heart in a diastolic phase;
FIG. 2 illustrates a cutaway view of the human heart in a systolic phase;
FIG. 3 illustrates a cutaway view of the human heart in a systolic phase showing mitral regurgitation;
FIG. 4 is the cutaway view of FIG. 3 annotated to illustrate a natural shape of mitral valve leaflets in the systolic phase;
FIG. 5 illustrates a healthy mitral valve with the leaflets closed as viewed from an atrial side of the mitral valve;
FIG. 6 illustrates a dysfunctional mitral valve with a visible gap between the leaflets as viewed from an atrial side of the mitral valve;
FIG. 7 illustrates a tricuspid valve viewed from an atrial side of the tricuspid valve;
FIGS. 8-14 show an example of an implantable device or implant, in various stages of deployment;
FIG. 15 shows an example of an implantable device or implant that is similar to the device illustrated by FIGS. 8-14, but where the paddles are independently controllable;
FIGS. 16-21 show the example implantable device or implant of FIGS. 8-14 being delivered and implanted within a native valve;
FIG. 22 shows a perspective view of an example implantable device or implant in a closed position;
FIG. 23 shows a perspective view of an example implantable device or implant in a closed position;
FIG. 24 illustrates an example valve repair device with paddles in an open position;
FIG. 25A illustrates another example valve repair device with paddles in a closed position;
FIG. 25B illustrates a top view of an example valve repair device;
FIG. 26 illustrates a perspective view of an example implantable device having paddles of adjustable widths;
FIG. 27 is a cross-section of the implantable device of FIG. 26 in which the implantable device is bisected;
FIG. 28 is another cross-section of the implantable device of FIG. 26 in which the implantable device is bisected along a plane perpendicular to the plane illustrated in FIG. 28;
FIG. 29 is a schematic illustration of an example implant catheter assembly coupled to an implantable device in which an actuation element is coupled to a paddle actuation control and to a driver head of the implantable device;
FIG. 30 is an illustration of the assembly of FIG. 29 with the implantable device rotated 90 degrees to show the paddle width adjustment element coupled to an inner end of the connector of the implantable device and coupled to a paddle width control;
FIG. 31 is an illustration of an example an interface between a connector and outer frame portions of an implantable device having an adjustable paddle width;
FIG. 32 is a side view of an example interface including a plurality of sutures extending through eyelets of a connector and outer frame portions of an implantable device having an adjustable paddle width;
FIG. 33 is a perspective illustration of the example interface of FIG. 32;
FIG. 34A is an illustration of an example of an interface including a first suture extending through eyelets of a connector and outer frame portions and through a connecting anchor of the connector of an implantable device;
FIG. 34B is an illustration of an example of an interface in which a second suture extending through eyelets of a connector and outer frame portions and through a connecting anchor of the connector has been added to the interface of FIG. 34A;
FIG. 34C is an illustration of an example of an interface in which a third suture extending through eyelets of a connector and outer frame portions has been added to the interface of FIG. 34B;
FIG. 34D is an illustration of an example of an interface in which a fourth suture extending through eyelets of a connector and outer frame portions has been added to the interface of FIG. 34C;
FIG. 35A is an illustration of an example of an interface including a first suture extending through eyelets of a connector and outer frame portions and through a connecting anchor of the connector of an implantable device;
FIG. 35B is an illustration of an example of an interface in which ends of the suture FIG. 34A are threaded through the eyelets of the connector a second time;
FIG. 35C is an illustration of an example of an interface in which one end of the suture extending through eyelets of a connector in FIG. 35B is threaded through one or more sleeves surrounding the outer frame portions and the connector;
FIGS. 35D-35I illustrate of an example of an interface including three sutures that connect a connector to outer frame portions of an implantable device;
FIG. 36 is an illustration of an example of a distal end of an implantable device including an actuation cap having a rounded portion extending between a retaining portion of the implantable device and a connector of the paddles of the implantable device;
FIG. 37 is an illustration of an example of a distal end of an implantable device including an actuation cap having one or more flexible portions extending between a retaining portion of the implantable device and a connector of the paddles of the implantable device; and
FIG. 38 is an illustration of an example of a distal end of an implantable device including a biasing element positioned between a retaining portion of the implantable device and an actuation cap.

### DETAILED DESCRIPTION

The following description refers to the accompanying drawings, which illustrate implementations of the present disclosure. Other implementations having different structures and operation do not depart from the scope of the present disclosure.

Implementations of the present disclosure are directed to systems, devices, methods, etc. for repairing a defective heart valve. For example, implementations of valve repair devices, implantable devices, implants, and systems (including systems for delivery thereof) are disclosed herein, and any combination of these options can be made unless specifically excluded. In other words, individual components of the disclosed devices and systems can be combined unless mutually exclusive or otherwise physically impossible. Further, the technique(s) and method(s) herein can be performed on a living animal or on a simulation, such as on a cadaver, cadaver heart, simulator (e.g., with the body parts, heart, tissue, etc. being simulated), etc. The term "simulation" encompass encompasses performing a method on a cadaver, a computer simulator, an imaginary person (e.g., demonstrating in the air on an imaginary heart), etc.

As described herein, when one or more components are described as being connected, joined, affixed, coupled, attached, or otherwise interconnected, such interconnection can be direct as between the components or can be indirect such as through the use of one or more intermediary components. Also as described herein, reference to a "member," "component," or "portion" shall not be limited to a single structural member, component, or element but can include an assembly of components, members, or elements. Also as described herein, the terms "substantially" and "about" are defined as at least close to (and includes) a given value or state (preferably within 10% of, more preferably within 1% of, and most preferably within 0.1% of).

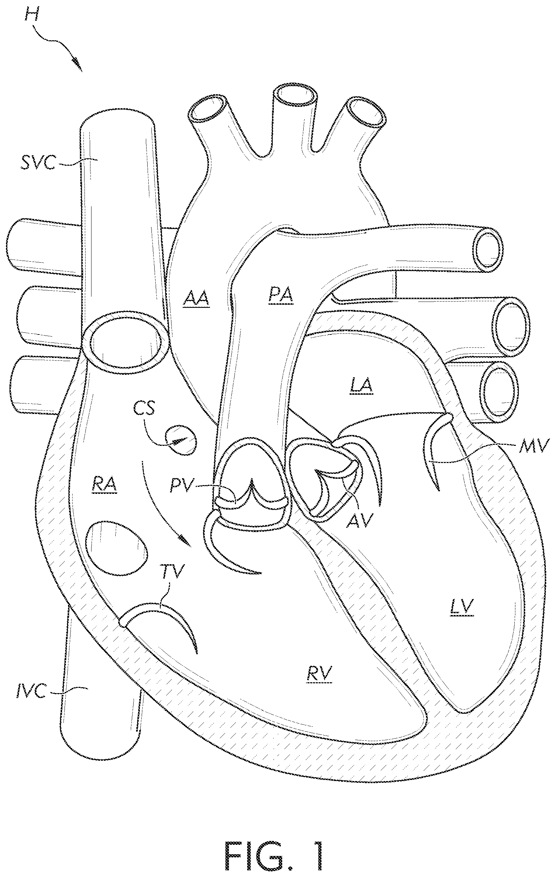

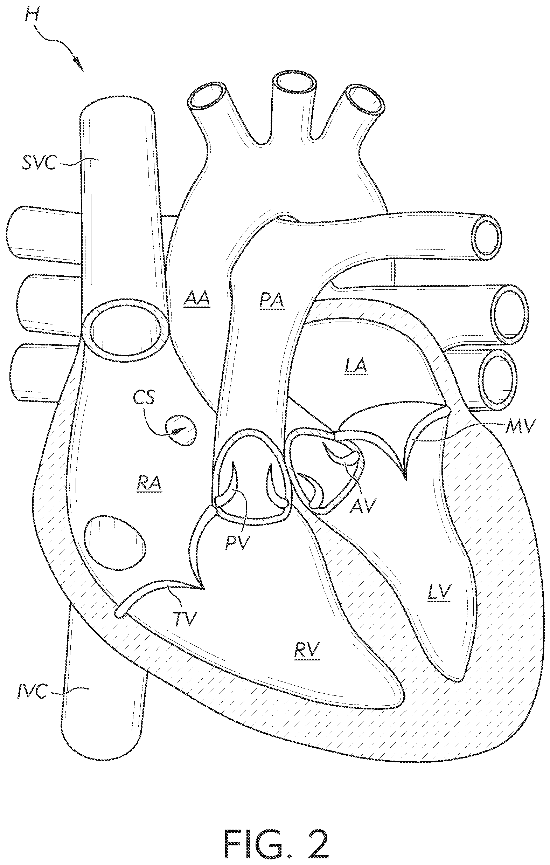

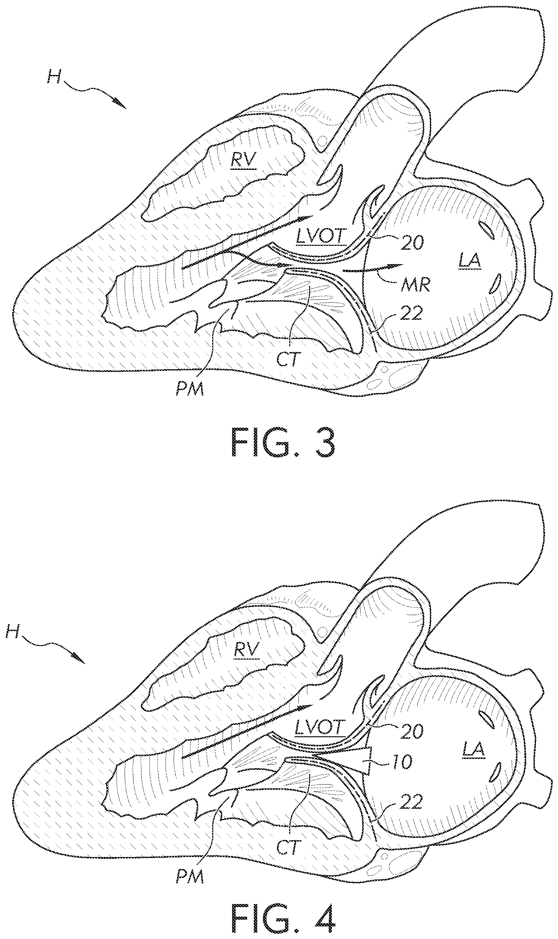

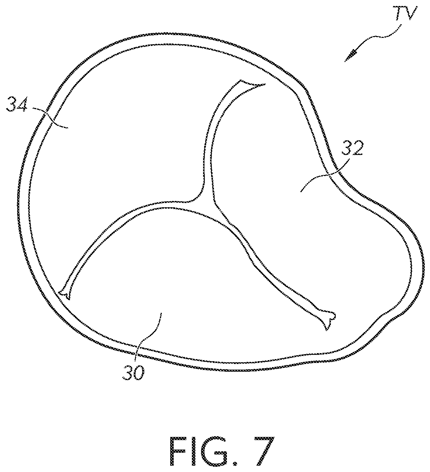

FIGS. 1 and 2 are cutaway views of the human heart H in diastolic and systolic phases, respectively. The right ventricle RV and left ventricle LV are separated from the right atrium RA and left atrium LA, respectively, by the tricuspid valve TV and mitral valve MV; i.e., the atrioventricular valves. Additionally, the aortic valve AV separates the left ventricle LV from the ascending aorta AA, and the pulmonary valve PV separates the right ventricle from the pulmonary artery PA. Each of these valves has flexible leaflets (e.g., leaflets 20, 22 shown in FIGS. 3-6 and leaflets 30, 32, 34 shown in FIG. 7) extending inward across the respective orifices that come together or "coapt" in the flow stream to form the one-way, fluid-occluding surfaces. The native valve repair systems of the present application are frequently described and/or illustrated with respect to the mitral valve MV. Therefore, anatomical structures of the left atrium LA and left ventricle LV will be explained in greater detail. However, the devices described herein can also be used in repairing other native valves, e.g., the devices can be used in repairing the tricuspid valve TV, the aortic valve AV, and the pulmonary valve PV.

The left atrium LA receives oxygenated blood from the lungs. During the diastolic phase, or diastole, seen in FIG. 1, the blood that was previously collected in the left atrium LA (during the systolic phase) moves through the mitral valve MV and into the left ventricle LV by expansion of the left ventricle LV. In the systolic phase, or systole, seen in FIG. 2, the left ventricle LV contracts to force the blood through the aortic valve AV and ascending aorta AA into the body. During systole, the leaflets of the mitral valve MV close to prevent the blood from regurgitating from the left ventricle LV and back into the left atrium LA and blood is collected in the left atrium from the pulmonary vein. In some implementations, the devices described by the present application are used to repair the function of a defective mitral valve MV. That is, the devices are configured to help close the leaflets of the mitral valve to prevent, inhibit or reduce blood from regurgitating from the left ventricle LV and back into the left atrium LA. Many of the devices described in the present application are designed to easily grasp and secure the native leaflets around a coaptation element or spacer that beneficially acts as a filler in the regurgitant orifice to prevent or inhibit back flow or regurgitation during systole, though this is not necessary.

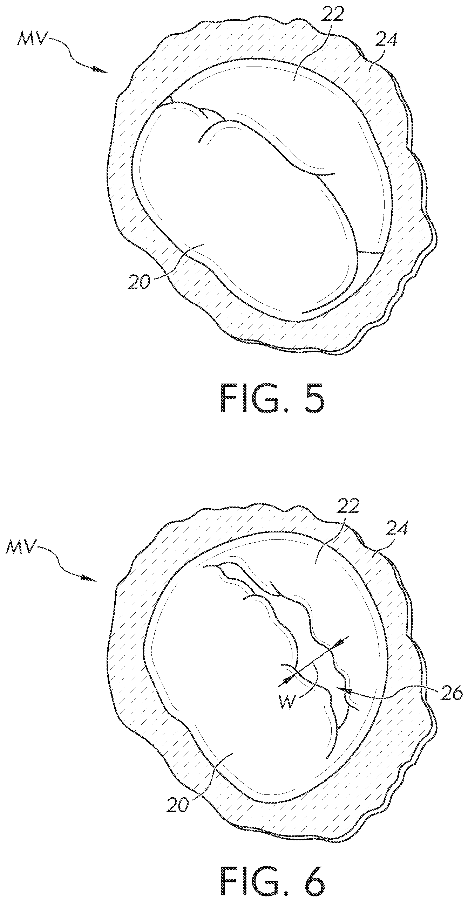

Referring now to FIGS. 1-7, the mitral valve MV includes two leaflets, the anterior leaflet 20 and the posterior leaflet 22. The mitral valve MV also includes an annulus 24 (see Fig. 5), which is a variably dense fibrous ring of tissues that encircles the leaflets 20, 22. Referring to FIGS. 3 and 4, the mitral valve MV is anchored to the wall of the left ventricle LV by chordae tendineae CT. The chordae tendineae CT are cord-like tendons that connect the papillary muscles PM (i.e., the muscles located at the base of the chordae tendineae CT and within the walls of the left ventricle LV) to the leaflets 20, 22 of the mitral valve MV. The papillary muscles PM serve to limit the movements of leaflets 20, 22 of the mitral valve MV and prevent the mitral valve MV from being reverted. The mitral valve MV opens and closes in response to pressure changes in the left atrium LA and the left ventricle LV. The papillary muscles PM do not open or close the mitral valve MV. Rather, the papillary muscles PM support or brace the leaflets 20, 22 against the high pressure needed to circulate blood throughout the body. Together the papillary muscles PM and the chordae tendineae CT are known as the subvalvular apparatus, which functions to keep the mitral valve MV from prolapsing into the left atrium LA when the mitral valve closes. As seen from a Left Ventricular Outflow Tract (LVOT) view shown in FIG. 3, the anatomy of the leaflets 20, 22 is such that the inner sides of the leaflets coapt at the free end portions and the leaflets 20, 22 start receding or spreading apart from each other. The leaflets 20, 22 spread apart in the atrial direction, until each leaflet meets with the mitral annulus.

Various disease processes can impair proper function of one or more of the native valves of the heart H. These disease processes include degenerative processes (e.g., Barlow's Disease, fibroelastic deficiency, etc.), inflammatory processes (e.g., Rheumatic Heart Disease), and infectious processes (e.g., endocarditis, etc.). In addition, damage to the left ventricle LV or the right ventricle RV from prior heart attacks (i.e., myocardial infarction secondary to coronary artery disease) or other heart diseases (e.g., cardiomyopathy, etc.) may distort a native valve's geometry, which may cause the native valve to dysfunction. However, the majority of patients undergoing valve surgery, such as surgery to the mitral valve MV, suffer from a degenerative disease that causes a malfunction in a leaflet (e.g., leaflets 20, 22) of a native valve (e.g., the mitral valve MV), which results in prolapse and regurgitation.

Generally, a native valve may malfunction in different ways: including (1) valve stenosis; and (2) valve regurgitation. Valve stenosis occurs when a native valve does not open completely and thereby causes an obstruction of blood flow. Typically, valve stenosis results from buildup of calcified material on the leaflets of a valve, which causes the leaflets to thicken and impairs the ability of the valve to fully open to permit forward blood flow. Valve regurgitation occurs when the leaflets of the valve do not close completely thereby causing blood to leak back into the prior chamber (e.g., causing blood to leak from the left ventricle to the left atrium).

There are three main mechanisms by which a native valve becomes regurgitant-or incompetent-which include Carpentier's type I, type II, and type III malfunctions. A Carpentier type I malfunction involves the dilation of the annulus such that normally functioning leaflets are distracted from each other and fail to form a tight seal (i.e., the leaflets do not coapt properly). Included in a type I mechanism malfunction are perforations of the leaflets, as are present in endocarditis. A Carpentier's type II malfunction involves prolapse of one or more leaflets of a native valve above a plane of coaptation. A Carpentier's type III malfunction involves restriction of the motion of one or more leaflets of a native valve such that the leaflets are abnormally constrained below the plane of the annulus. Leaflet restriction may be caused by rheumatic disease or dilation of a ventricle.

Referring to FIG. 5, when a healthy mitral valve MV is in a closed position, the anterior leaflet 20 and the posterior leaflet 22 coapt, which prevents blood from leaking from the left ventricle LV to the left atrium LA. Referring to FIGS. 3 and 6, mitral regurgitation MR occurs when the anterior leaflet 20 and/or the posterior leaflet 22 of the mitral valve MV is displaced into the left atrium LA during systole so that the edges of the leaflets 20, 22 are not in contact with each other. This failure to coapt causes a gap 26 between the anterior leaflet 20 and the posterior leaflet 22, which allows blood to flow back into the left atrium LA from the left ventricle LV during systole, as illustrated by the mitral regurgitation MR flow path shown in FIG. 3. Referring to FIG. 6, the gap 26 can have a width W between about 2.5 mm and about 17.5 mm, between about 5 mm and about 15 mm, between about 7.5 mm and about 12.5 mm, or about 10 mm. In some situations, the gap 26 can have a width W greater than 15 mm or even 17.5 mm. As set forth above, there are several different ways that a leaflet (e.g., leaflets 20, 22 of mitral valve MV) may malfunction which can thereby lead to valvular regurgitation.

In any of the above-mentioned situations, a valve repair device or implant is desired that is capable of engaging the anterior leaflet 20 and the posterior leaflet 22 to close the gap 26 and prevent or inhibit regurgitation of blood through the mitral valve MV. As can be seen in FIG. 4, an abstract representation of an implantable device, valve repair device, or implant 10 is shown implanted between the leaflets 20, 22 such that regurgitation does not occur during systole (compare FIG. 3 with FIG. 4). In some implementations, the coaptation element (e.g., spacer, coaption element, gap filler, etc.) of the device 10 has a generally tapered or triangular shape that naturally adapts to the native valve geometry and to its expanding leaflet nature (toward the annulus). In this application, the terms spacer, coaption element, coaptation element, and gap filler are used interchangeably and refer to an element that fills a portion of the space between native valve leaflets and/or that is configured such that the native valve leaflets engage or "coapt" against (e.g., such that the native leaflets coapt against the coaption element, coaptation element, spacer, etc. instead of only against one another).

Although stenosis or regurgitation may affect any valve, stenosis is predominantly found to affect either the aortic valve AV or the pulmonary valve PV, and regurgitation is predominantly found to affect either the mitral valve MV or the tricuspid valve TV. Both valve stenosis and valve regurgitation increase the workload of the heart H and may lead to very serious conditions if left un-treated; such as endocarditis, congestive heart failure, permanent heart damage, cardiac arrest, and ultimately death. Because the left side of the heart (i.e., the left atrium LA, the left ventricle LV, the mitral valve MV, and the aortic valve AV) are primarily responsible for circulating the flow of blood throughout the body. Accordingly, because of the substantially higher pressures on the left side heart dysfunction of the mitral valve MV or the aortic valve AV is particularly problematic and often life threatening.

Malfunctioning native heart valves may either be repaired or replaced. Repair typically involves the preservation and correction of the patient's native valve. Replacement typically involves replacing the patient's native valve with a biological or mechanical substitute. Typically, the aortic valve AV and pulmonary valve PV are more prone to stenosis. Because stenotic damage sustained by the leaflets is irreversible, treatments for a stenotic aortic valve or stenotic pulmonary valve can be removal and replacement of the valve with a surgically implanted heart valve, or displacement of the valve with a transcatheter heart valve. The mitral valve MV and the tricuspid valve TV are more prone to deformation of leaflets and/or surrounding tissue, which, as described above, may prevent the mitral valve MV or tricuspid valve TV from closing properly and allows for regurgitation or back flow of blood from the ventricle into the atrium (e.g., a deformed mitral valve MV may allow for regurgitation or back flow from the left ventricle LV to the left atrium LA as shown in FIG. 3). The regurgitation or back flow of blood from the ventricle to the atrium results in valvular insufficiency. Deformations in the structure or shape of the mitral valve MV or the tricuspid valve TV are often repairable. In addition, regurgitation may occur due to the chordae tendineae CT becoming dysfunctional (e.g., the chordae tendineae CT may stretch or rupture), which allows the anterior leaflet 20 and the posterior leaflet 22 to be reverted such that blood is regurgitated into the left atrium LA. The problems occurring due to dysfunctional chordae tendineae CT can be repaired by repairing the chordae tendineae CT or the structure of the mitral valve MV (e.g., by securing the leaflets 20, 22 at the affected portion of the mitral valve).

The devices and procedures disclosed herein often make reference to repairing the structure of a mitral valve. However, it should be understood that the devices and concepts provided herein can be used to repair any native valve, as well as any component of a native valve. Such devices can be used between the leaflets 20, 22 of the mitral valve MV to prevent or inhibit regurgitation of blood from the left ventricle into the left atrium. With respect to the tricuspid valve TV (FIG. 7), any of the devices and concepts herein can be used between any two of the anterior leaflet 30, septal leaflet 32, and posterior leaflet 34 to prevent or inhibit regurgitation of blood from the right ventricle into the right atrium. In addition, any of the devices and concepts provided herein can be used on all three of the leaflets 30, 32, 34 together to prevent or inhibit regurgitation of blood from the right ventricle to the right atrium. That is, the valve repair devices or implants provided herein can be centrally located between the three leaflets 30, 32, 34.

An example implantable device or implant can optionally have a coaptation element (e.g., spacer, coaption element, gap filler, etc.) and at least one anchor (e.g., one, two, three, or more). In some implementations, an implantable device or implant can have any combination or sub-combination of the features disclosed herein without a coaptation element. When included, the coaptation element (e.g., spacer, coaption element, gap filler, etc.) is configured to be positioned within the native heart valve orifice to help fill the space between the leaflets and form a more effective seal, thereby reducing or preventing or inhibiting regurgitation described above. The coaptation element can have a structure that is impervious to blood (or that resists blood flow therethrough) and that allows the native leaflets to close around the coaptation element during ventricular systole to block blood from flowing from the left or right ventricle back into the left or right atrium, respectively. The device or implant can be configured to seal against two or three native valve leaflets; that is, the device can be used in the native mitral (bicuspid) and tricuspid valves. The coaptation element is sometimes referred to herein as a spacer because the coaptation element can fill a space between improperly functioning native leaflets (e.g., mitral leaflets 20, 22 or tricuspid leaflets 30, 32, 34) that do not close completely.

The optional coaptation element (e.g., spacer, coaptation element, gap filler, etc.) can have various shapes. In some implementations, the coaptation element can have an elongated cylindrical shape having a round cross-sectional shape. In some implementations, the coaptation element can have an oval cross-sectional shape, an ovoid cross-sectional shape, a crescent cross-sectional shape, a rectangular cross-sectional shape, or various other non-cylindrical shapes. In some implementations, the coaptation element can have an atrial portion positioned in or adjacent to the atrium, a ventricular or lower portion positioned in or adjacent to the ventricle, and a side surface that extends between the native leaflets. In some implementations configured for use in the tricuspid valve, the atrial or upper portion is positioned in or adjacent to the right atrium, and the ventricular or lower portion is positioned in or adjacent to the right ventricle, and the side surfaces extend between the native tricuspid leaflets.

In some implementations, the anchor can be configured to secure the device to one or both of the native leaflets such that the coaptation element is positioned between the two native leaflets. In some implementations configured for use in the tricuspid valve, the anchor is configured to secure the device to one, two, or three of the tricuspid leaflets such that the coaptation element is positioned between the three native leaflets. In some implementations, the anchor can attach to the coaptation element at a location adjacent the ventricular portion of the coaptation element. In some implementations, the anchor can attach to an actuation element (e.g., an actuation shaft, actuation tube, actuation wire, etc.) to which the coaptation element is also attached. In some implementations, the anchor and the coaptation element can be positioned independently with respect to each other by separately moving each of the anchor and the coaptation element along the longitudinal axis of the actuation element (e.g., actuation shaft, actuation rod, actuation tube, actuation wire, etc.). In some implementations, the anchor and the coaptation element can be positioned simultaneously by moving the anchor and the coaptation element together along the longitudinal axis of the actuation element (e.g., shaft, actuation wire, etc.). The anchor can be configured to be positioned behind a native leaflet when implanted such that the leaflet is grasped by the anchor.

The device or implant can be configured to be implanted via a delivery system or other means for delivery. The delivery system can comprise one or more of a guide/delivery sheath, a delivery catheter, a steerable catheter, an implant catheter, tube, combinations of these, etc. The coaptation element and the anchor can be compressible to a radially compressed state and can be self-expandable to a radially expanded state when compressive pressure is released. The device can be configured for the anchor to be expanded radially away from the still-compressed coaptation element initially in order to create a gap between the coaptation element and the anchor. A native leaflet can then be positioned in the gap. The coaptation element can be expanded radially, closing the gap between the coaptation element and the anchor and capturing the leaflet between the coaptation element and the anchor. In some implementations, the anchor and coaptation element are optionally configured to self-expand. The implantation methods can be different and are more fully discussed below with respect to each implementation. Additional information regarding these and other delivery methods can be found in U.S. Pat. No. 8,449,599 and U.S. Patent Application Publication Nos. 2014/0222136, 2014/0067052, 2016/0331523, and PCT patent application publication Nos. WO2020/076898. These method(s) can be performed on a living animal or on a simulation, such as on a cadaver, cadaver heart, imaginary person, simulator (e.g., with the body parts, heart, tissue, etc. being simulated), etc. *mutatis mutandis.*

The disclosed devices or implants can be configured such that the anchor is connected to a leaflet, taking advantage of the tension from native chordae tendineae to resist high systolic pressure urging the device toward the left atrium. During diastole, the devices can rely on the compressive and retention forces exerted on the leaflet that is grasped by the anchor.

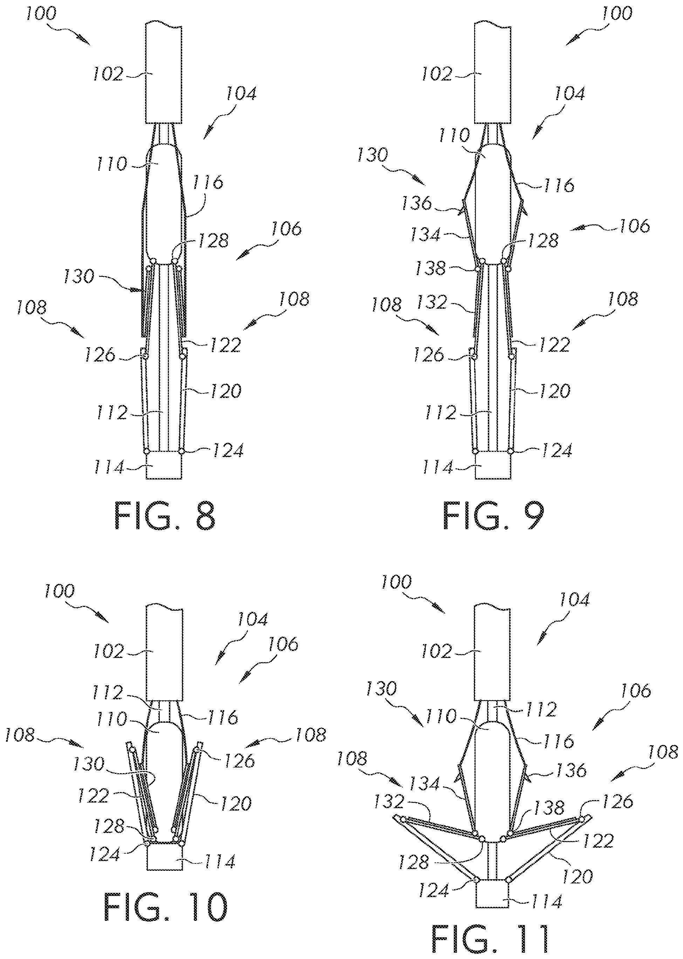

Referring now to FIGS. 8-15, a schematically illustrated implantable device or implant 100 (e.g., an implantable prosthetic device, a prosthetic spacer device, a valve repair device, etc.) is shown in various stages of deployment. The device or implant 100 and other similar devices/implants are described in more detail in PCT patent application publication Nos. WO2018/195215, WO2020/076898, and WO 2019/139904. The device 100 can include any other features for an implantable device or implant discussed in the present application or the applications cited above, and the device 100 can be positioned to engage valve tissue (e.g., leaflets 20, 22, 30, 32, 34) as part of any suitable valve repair system (e.g., any valve repair system disclosed in the present application or the applications cited above).

The device or implant 100 is deployed from a delivery system 102. The delivery system 102 can comprise one or more of a catheter, a sheath, a guide catheter/sheath, a delivery catheter/sheath, a steerable catheter, an implant catheter, a tube, a channel, a pathway, combinations of these, etc. The device or implant 100 includes a coaptation portion 104 and an anchor portion 106.

In some implementations, the coaptation portion 104 of the device or implant 100 includes a coaptation element 110 that is adapted to be implanted between leaflets of a native valve (e.g., a native mitral valve, native tricuspid valve, etc.) and is slidably attached to an actuation element 112 (e.g., actuation wire, actuation shaft, actuation tube, etc.). The anchor portion 106 includes one or more anchors 108 that are actuatable between open and closed conditions and can take a wide variety of forms, such as, for example, paddles, gripping elements, or the like. Actuation of the actuation element 112 opens and closes the anchor portion 106 of the device 100 to grasp the native valve leaflets during implantation. The actuation element 112 (as well as other actuation elements disclosed herein) can take a wide variety of different forms (e.g., as a wire, rod, shaft, tube, screw, suture, line, strip, combination of these, etc.), be made of a variety of different materials, and have a variety of configurations. As one example, the actuation element can be threaded such that rotation of the actuation element moves the anchor portion 106 relative to the coaptation portion 104. Or, the actuation element can be unthreaded, such that pushing or pulling the actuation element 112 moves the anchor portion 106 relative to the coaptation portion 104.

The anchor portion 106 and/or anchors of the device 100 include outer paddles 120 and inner paddles 122 that are, in some implementations, connected between a cap 114 and a coaptation element 110 by portions 124, 126, 128. The portions 124, 126, 128 can be jointed and/or flexible to move between all of the positions described below. The interconnection of the outer paddles 120, the inner paddles 122, the coaptation element 110, and the cap 114 by the portions 124, 126, and 128 can constrain the device to the positions and movements illustrated herein.

In some implementations, the delivery system 102 includes a steerable catheter, implant catheter, and the actuation element 112 (e.g., actuation wire, actuation shaft, etc.). These can be configured to extend through a guide catheter/sheath (e.g., a transseptal sheath, etc.). In some implementations, the actuation element 112 extends through a delivery catheter and the coaptation element 110 to the distal end (e.g., a cap 114 or other attachment portion at the distal connection of the anchor portion 106). Extending and retracting the actuation element 112 increases and decreases the spacing between the coaptation element 110 and the distal end of the device (e.g., the cap 114 or other attachment portion), respectively. In some implementations, a collar or other attachment element removably attaches the coaptation element 110 to the delivery system 102, either directly or indirectly, so that the actuation element 112 slides through the collar or other attachment element and, in some implementations, through a coaptation element 110 during actuation to open and close the paddles 120, 122 of the anchor portion 106 and/or anchors 108.

In some implementations, the anchor portion 106 and/or anchors 108 can include attachment portions or gripping members. The illustrated gripping members can comprise clasps 130 that include a base or fixed arm 132, a moveable arm 134, optional friction-enhancing elements, other securing structures 136 (e.g., barbs, protrusions, ridges, grooves, textured surfaces, adhesive, etc.), and a joint portion 138. The fixed arms 132 are attached to the inner paddles 122. In some implementations, the fixed arms 132 are attached to the inner paddles 122 with the joint portion 138 disposed proximate the coaptation element 110. The joint portion 138 provides a spring force between the fixed and moveable arms 132, 134 of the clasp 130. The joint portion 138 can be any suitable joint, such as a flexible joint, a spring joint, a pivot joint, or the like. In some implementations, the joint portion 138 is a flexible piece of material integrally formed with the fixed and moveable arms 132, 134. The fixed arms 132 are attached to the inner paddles 122 and remain stationary or substantially stationary relative to the inner paddles 122 when the moveable arms 134 are opened to open the clasps 130 and expose the optional barbs or other friction-enhancing elements 136 (e.g., ridges, roughened surface, etc.).

In some implementations, the clasps 130 are opened by applying tension to actuation lines 116 attached to the moveable arms 134, thereby causing the moveable arms 134 to articulate, flex, or pivot on the joint portions 138. The actuation lines 116 extend through the delivery system 102 (e.g., through a steerable catheter and/or an implant catheter). Other actuation mechanisms are also possible.

The actuation line 116 can take a wide variety of forms, such as, for example, a line, a suture, a wire, a rod, a catheter, or the like. The clasps 130 can be spring loaded so that in the closed position the clasps 130 continue to provide a pinching force on the grasped native leaflet. Optional barbs or other friction-enhancing elements 136 of the clasps 130 can grab, pinch, and/or pierce the native leaflets to further secure the native leaflets.

During implantation, the paddles 120, 122 can be opened and closed, for example, to grasp the native leaflets (e.g., native mitral valve leaflets, etc.) between the paddles 120, 122 and/or between the paddles 120, 122 and a coaptation element 110 (e.g., a spacer, plug, membrane, etc.). The clasps 130 can be used to grasp and/or further secure the native leaflets by engaging the leaflets with optional barbs or other friction-enhancing elements 136 and pinching the leaflets between the moveable and fixed arms 134, 132. The optional barbs or other friction-enhancing elements 136 (e.g., protrusions, ridges, grooves, textured surfaces, adhesive, etc.) of the clasps 130 increase friction with the leaflets or can partially or completely puncture the leaflets. The actuation lines 116 can be actuated separately so that each clasp 130 can be opened and closed separately. Separate operation allows one leaflet to be grasped at a time, or for the repositioning of a clasp 130 on a leaflet that was insufficiently grasped, without altering a successful grasp on the other leaflet. The clasps 130 can be opened and closed relative to the position of the inner paddle 122 (as long as the inner paddle is in an open or at least partially open position), thereby allowing leaflets to be grasped in a variety of positions as the particular situation requires.

Referring now to FIG. 8, the device 100 is shown in an elongated or fully open condition for deployment from an implant delivery catheter of the delivery system 102. The device 100 is disposed at the end of the catheter of the delivery system 102 in the fully open position. In the elongated condition the cap 114 is spaced apart from the coaptation element 110 such that the paddles 120, 122 are fully extended. In some implementations, an angle formed between the interior of the outer and inner paddles 120, 122 is approximately 180 degrees. The clasps 130 can be kept in a closed condition during deployment through the delivery system. The actuation lines 116 can extend and attach to the moveable arms 134.

Referring now to FIG. 9, the device 100 is shown in an elongated condition, similar to FIG. 8, but with the clasps 130 in a fully open position, ranging from about 140 degrees to about 200 degrees, from about 170 degrees to about 190 degrees, or about 180 degrees between fixed and moveable portions 132, 134 of the clasps 130.

Referring now to FIG. 10, the device 100 is shown in a shortened or fully closed condition. To move the device 100 from the elongated condition to the shortened condition, the actuation element 112 is retracted to pull the cap 114 towards the coaptation element 110. The connection portion(s) 126 (e.g., joint(s), flexible connection(s), etc.) between the outer paddle 120 and inner paddle 122 are constrained in movement such that compression forces acting on the outer paddle 120 from the cap 114 being retracted towards the coaptation element 110 cause the paddles or gripping elements to move radially outward. During movement from the open position to the closed position, the outer paddles 120 maintain an acute angle with the actuation element 112. The outer paddles 120 can optionally be biased toward a closed position. The inner paddles 122 during the same motion move through a considerably larger angle as they are oriented away from the coaptation element 110 in the open condition and collapse along the sides of the coaptation element 110 in the closed condition.

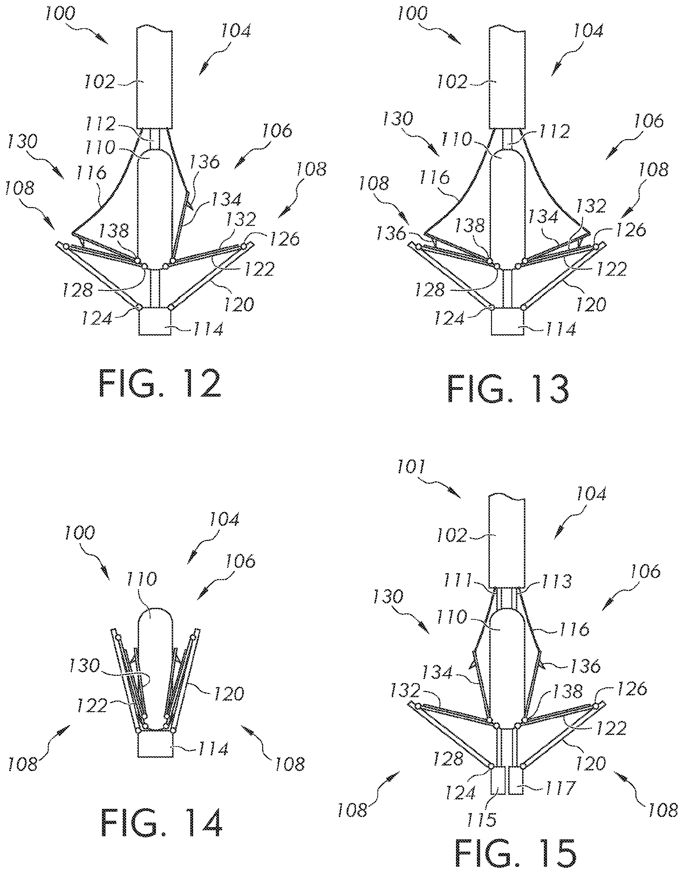

Referring now to FIGS. 11-13, the device 100 is shown in a partially open, grasp-ready condition. To transition from the fully closed to the partially open condition, the actuation element (e.g., actuation wire, actuation shaft, etc.) is extended to push the cap 114 away from the coaptation element 110, thereby pulling on the outer paddles 120, which in turn pull on the inner paddles 122, causing the anchors or anchor portion 106 to partially unfold. The actuation lines 116 are also retracted to open the clasps 130 so that the leaflets can be grasped. In some implementations, the pair of inner and outer paddles 122, 120 are moved in unison, rather than independently, by a single actuation element 112. Also, the positions of the clasps 130 are dependent on the positions of the paddles 122, 120. For example, referring to FIG. 10 closing the paddles 122, 120 also closes the clasps. In some implementations, the paddles 120, 122 can be independently controllable. In the example illustrated by FIG. 15, the device 100 can have two actuation elements 111, 113 and two independent caps 115, 117 (or other attachment portions), such that one independent actuation element (e.g., wire, shaft, etc.) and cap (or other attachment portion) are used to control one paddle, and the other independent actuation element and cap (or other attachment portion) are used to control the other paddle.

Referring now to FIG. 12, one of the actuation lines 116 is extended to allow one of the clasps 130 to close. Referring now to FIG. 13, the other actuation line 116 is extended to allow the other clasp 130 to close. Either or both of the actuation lines 116 can be repeatedly actuated to repeatedly open and close the clasps 130.

Referring now to FIG. 14, the device 100 is shown in a fully closed and deployed condition. The delivery system 102 and actuation element 112 are retracted and the paddles 120, 122 and clasps 130 remain in a fully closed position. Once deployed, the device 100 can be maintained in the fully closed position with a mechanical latch or can be biased to remain closed through the use of spring materials, such as steel, other metals, plastics, composites, etc. or shape-memory alloys such as Nitinol. For example, the connection portions 124, 126, 128, the joint portions 138, and/or the inner and outer paddles 122, and/or an additional biasing component (not shown) can be formed of metals such as steel or shape-memory alloy, such as Nitinol-produced in a wire, sheet, tubing, or laser sintered powder-and are biased to hold the outer paddles 120 closed around the coaptation element 110 and the clasps 130 pinched around native leaflets. Similarly, the fixed and moveable arms 132, 134 of the clasps 130 are biased to pinch the leaflets. In some implementations, the attachment or connection portions 124, 126, 128, joint portions 138, and/or the inner and outer paddles 122, and/or an additional biasing component (not shown) can be formed of any other suitably elastic material, such as a metal or polymer material, to maintain the device 100 in the closed condition after implantation.

FIG. 15 illustrates an example where the paddles 120, 122 are independently controllable. The device 101 illustrated by FIG. 15 is similar to the device illustrated by FIG. 11, except the device 100 of FIG. 15 includes an actuation element that is configured as two independent actuation elements 111, 113 that are coupled to two independent caps 115, 117. To transition a first inner paddle 122 and a first outer paddle 120 from the fully closed to the partially open condition, the actuation element 111 is extended to push the cap 115 away from the coaptation element 110, thereby pulling on the outer paddle 120, which in turn pulls on the inner paddle 122, causing the first anchor 108 to partially unfold. To transition a second inner paddle 122 and a second outer paddle 120 from the fully closed to the partially open condition, the actuation element 113 is extended to push the cap 115 away from the spacer or coaptation element 110, thereby pulling on the outer paddle 120, which in turn pulls on the inner paddle 122, causing the second anchor 108 to partially unfold. The independent paddle control illustrated by FIG. 15 can be implemented on any of the devices disclosed by the present application. For comparison, in the example illustrated by FIG. 11, the pair of inner and outer paddles 122, 120 are moved in unison, rather than independently, by a single actuation element 112.

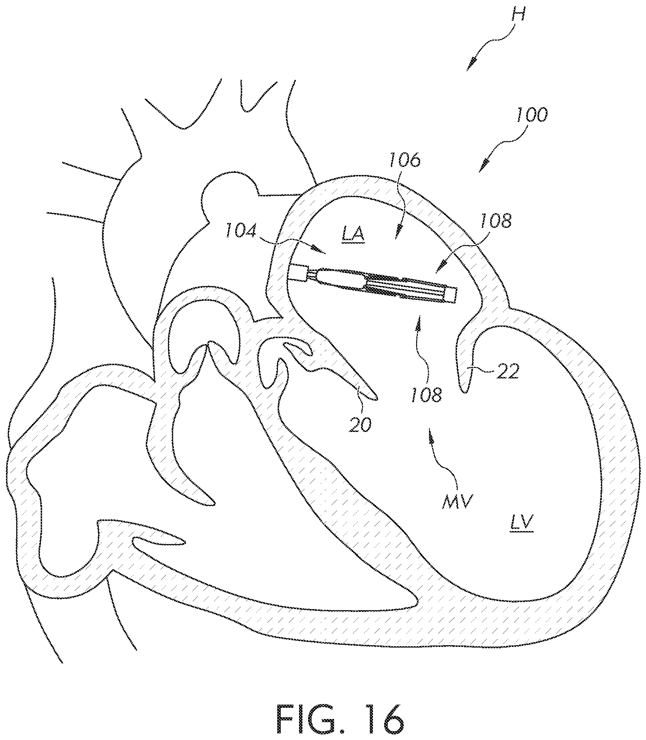

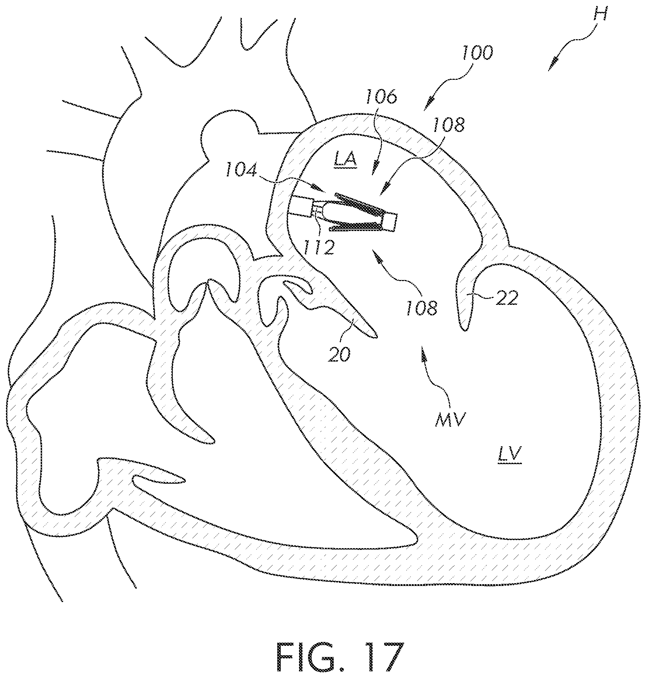

Referring now to FIGS. 16-21, the implantable device 100 of FIGS. 8-14 is shown being delivered and implanted within the native mitral valve MV of the heart H. Referring to FIG. 16, a delivery sheath/catheter is inserted into the left atrium LA through the septum and the implant/device 100 is deployed from the delivery catheter/sheath in the fully open condition as illustrated in FIG. 16. The actuation element 112 is then retracted to move the implant/device into the fully closed condition shown in FIG. 17.

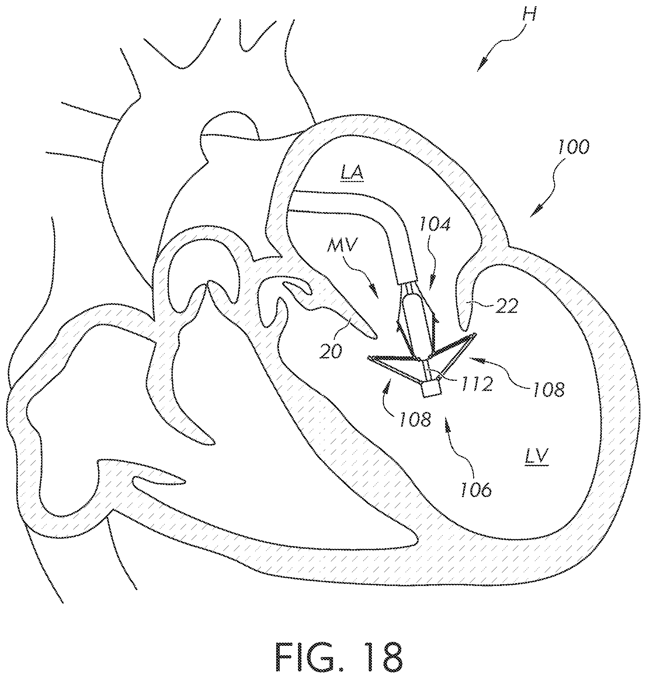

As can be seen in FIG. 18, the implant/device is moved into position within the mitral valve MV into the ventricle LV and partially opened so that the leaflets 20, 22 can be grasped. For example, a steerable catheter can be advanced and steered or flexed to position the steerable catheter as illustrated by FIG. 18. The implant catheter connected to the implant/device can be advanced from inside the steerable catheter to position the implant as illustrated by FIG. 18.

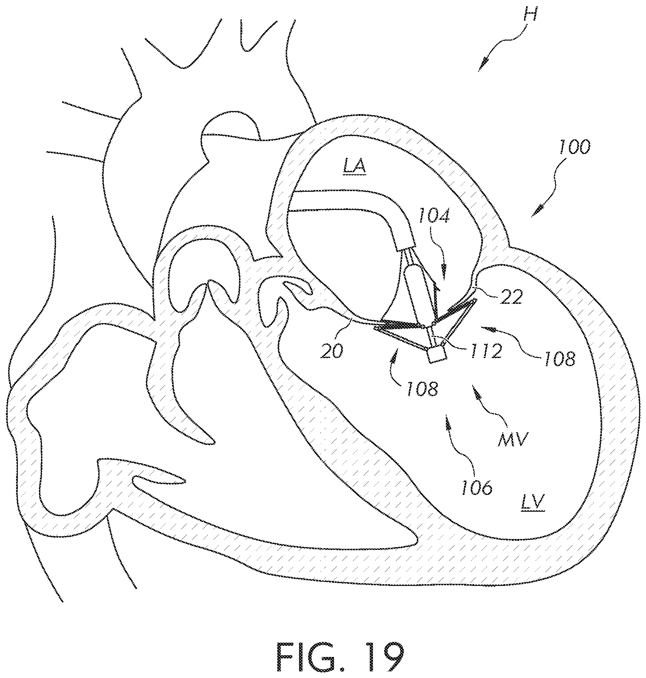

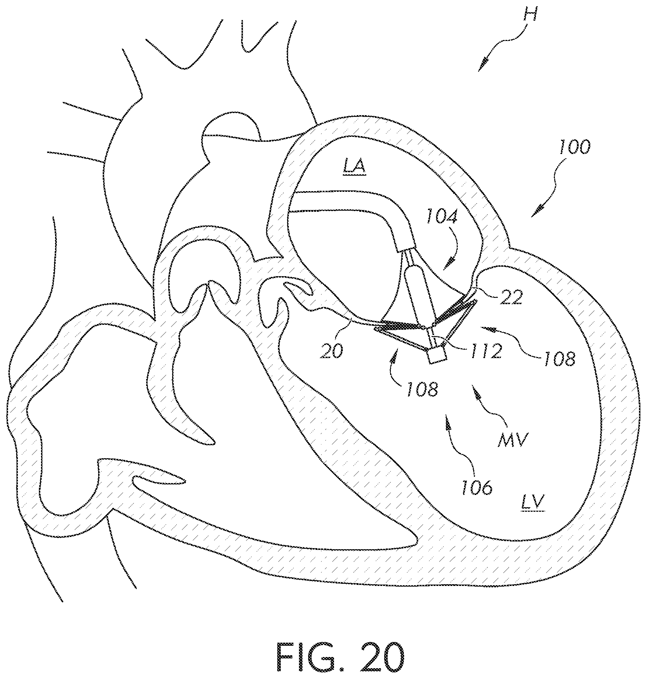

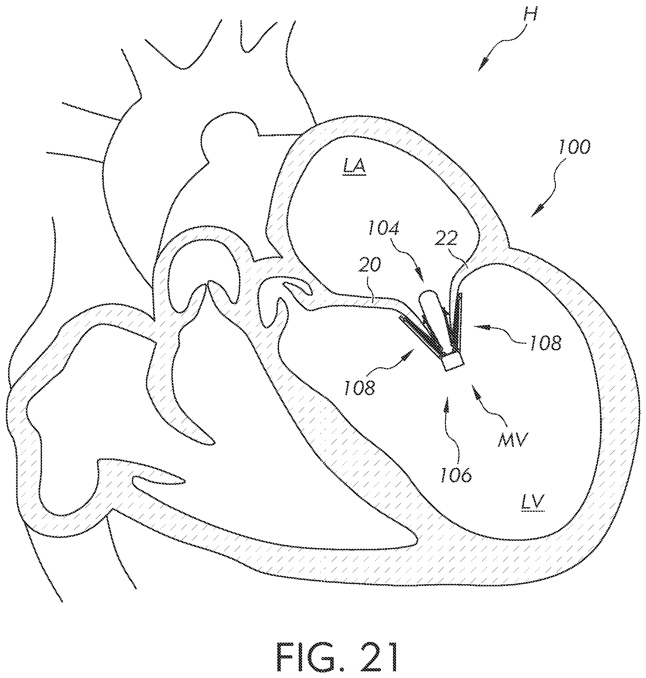

Referring now to FIG. 19, the implant catheter can be retracted into the steerable catheter to position the mitral valve leaflets 20, 22 in the clasps 130. An actuation line 116 is extended to close one of the clasps 130, capturing a leaflet 20. FIG. 20 shows the other actuation line 116 being then extended to close the other clasp 130, capturing the remaining leaflet 22. Lastly, as can be seen in FIG. 21, the delivery system 102 (e.g., steerable catheter, implant catheter, etc.), actuation element 112 and actuation lines 116 are then retracted and the device or implant 100 is fully closed and deployed in the native mitral valve MV.

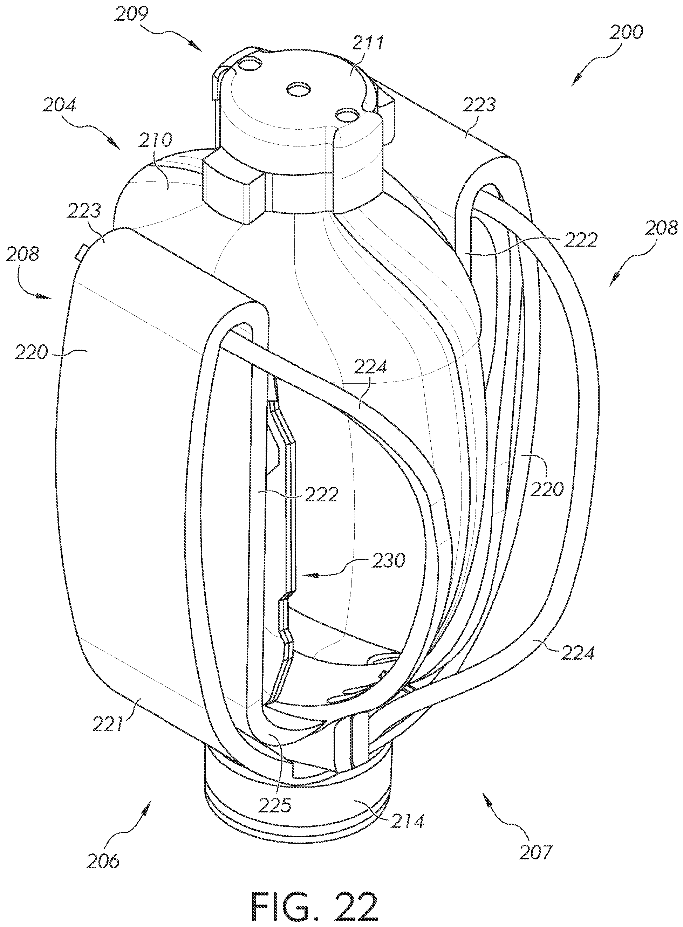

Any of the features disclosed by the present application can be used in a wide variety of different valve repair devices. FIGS. 22-24 illustrate examples of valve repair devices that can be modified to include any of the features disclosed by the present application. Any combination or sub-combination of the features disclosed by the present application can be combined with, substituted for, and/or added to any combination or sub-combination of the features of the valve repair devices illustrated by FIGS. 8-24.

Referring now to FIG. 22, an example of an implantable device or implant 200 is shown. The implantable device 200 is one of the many different configurations that the device 100 that is schematically illustrated in FIGS. 8-14 can take. The device 200 can include any other features for an implantable device or implant discussed in the present application, and the device 200 can be positioned to engage valve tissue 20, 22 as part of any suitable valve repair system (e.g., any valve repair system disclosed in the present application). The device/implant 200 can be a prosthetic spacer device, valve repair device, or another type of implant that attaches to leaflets of a native valve.

In some implementations, the implantable device or implant 200 includes a coaptation portion 204, a proximal or attachment portion 209, an anchor portion 206, and a distal portion 207. In some implementations, the coaptation portion 204 of the device optionally includes a coaptation element 210 (e.g., a spacer, coaption element, plug, membrane, sheet, gap filler, etc.) for implantation between leaflets of a native valve. In some implementations, the anchor portion 206 includes a plurality of anchors 208. The anchors can be configured in a variety of ways. In some implementations, each anchor 208 includes outer paddles 220, inner paddles 222, paddle extension members or paddle frames 224, and clasps 230. In some implementations, the attachment portion 209 includes a first or proximal collar 211 (or other attachment element) for engaging with a capture mechanism (e.g., a coupler, etc.) of a delivery system. A delivery system for the device 200 can be the same as or similar to delivery system 102 described above and can comprise one or more of a catheter, a sheath, a guide catheter/sheath, a delivery catheter/sheath, a steerable catheter, an implant catheter, a tube, a channel, a pathway, combinations of these, etc.

In some implementations, the coaptation element 210 and paddles 220, 222 are formed from a flexible material that can be a metal fabric, such as a mesh, woven, braided, or formed in any other suitable way or a laser cut or otherwise cut flexible material. The material can be cloth, shape-memory alloy wire-such as Nitinol-to provide shape-setting capability, or any other flexible material suitable for implantation in the human body.

An actuation element (e.g., actuation shaft, actuation rod, actuation tube, actuation wire, actuation line, etc.) can extend from a delivery system (not shown) to engage and enable actuation of the implantable device or implant 200. In some implementations, the actuation element extends through the proximal collar 211, and spacer or coaptation element 210 to engage a cap 214 of the distal portion 207. The actuation element can be configured to removably engage the cap 214 with a threaded connection, or the like, so that the actuation element can be disengaged and removed from the device 200 after implantation.

The coaptation element 210 extends from the proximal collar 211 (or other attachment element) to the inner paddles 222. In some implementations, the coaptation element 210 has a generally elongated and round shape, though other shapes and configurations are possible. In some implementations, the coaptation element 210 has an elliptical shape or cross-section when viewed from above and has a tapered shape or cross-section when seen from a front view and a round shape or cross-section when seen from a side view. A blend of these three geometries can result in the three-dimensional shape of the illustrated coaptation element 210 that achieves the benefits described herein. The round shape of the coaptation element 210 can also be seen, when viewed from above, to substantially follow or be close to the shape of the paddle frames 224.

The size and/or shape of the coaptation element 210 can be selected to minimize the number of implants that a single patient will require (preferably one), while at the same time maintaining low transvalvular gradients. In some implementations, the anterior-posterior distance at the top of the coaptation element is about 5 mm, and the medial-lateral distance of the coaptation element at its widest is about 10 mm. In some implementations, the overall geometry of the device 200 can be based on these two dimensions and the overall shape strategy described above. It should be readily apparent that the use of other anterior-posterior distance anterior-posterior distance and medial-lateral distance as starting points for the device will result in a device having different dimensions. Further, using other dimensions and the shape strategy described above will also result in a device having different dimensions.

In some implementations, the outer paddles 220 are jointably attached to the cap 214 of the distal portion 207 by connection portions 221 and to the inner paddles 222 by connection portions 223. The inner paddles 222 are jointably attached to the coaptation element by connection portions 225. In this manner, the anchors 208 are configured similar to legs in that the inner paddles 222 are like upper portions of the legs, the outer paddles 220 are like lower portions of the legs, and the connection portions 223 are like knee portions of the legs.

In some implementations, the inner paddles 222 are stiff, relatively stiff, rigid, have rigid portions and/or are stiffened by a stiffening member (e.g., bar, plate, stacked layers, etc.) or a fixed portion of the clasps 230. The inner paddle 222, the outer paddle 220, and the coaptation element can all be interconnected as described herein.

In some implementations, the paddle frames 224 are attached to the cap 214 at the distal portion 207 and extend to the connection portions 223 between the inner and outer paddles 222, 220. In some implementations, the paddle frames 224 are formed of a material that is more rigid and stiff than the material forming the paddles 222, 220 so that the paddle frames 224 provide support for the paddles 222, 220.

The paddle frames 224 can provide additional pinching force between the inner paddles 222 and the coaptation element 210 and assist in wrapping the leaflets around the sides of the coaptation element 210. That is, the paddle frames 224 can be configured with a round three-dimensional shape extending from the cap 214 to the connection portions 223 of the anchors 208. The connections between the paddle frames 224, the outer and inner paddles 220, 222, the cap 214, and the coaptation element 210 can constrain each of these parts to the movements and positions described herein. In particular the connection portion 223 is constrained by its connection between the outer and inner paddles 220, 222 and by its connection to the paddle frame 224. Similarly, the paddle frame 224 is constrained by its attachment to the connection portion 223 (and thus the inner and outer paddles 222, 220) and to the cap 214.

The wide configuration of the paddle frames 224 provides increased surface area compared to the inner paddles 222 alone. The increased surface area can distribute the clamping force of the paddles 220 and paddle frames 224 against the native leaflets over a relatively larger surface of the native leaflets in order to further protect the native leaflet tissue.

Additional features of the device 200, modified versions of the device, delivery systems for the device, and methods for using the device and delivery system are disclosed by Patent Cooperation Treaty International Application No. PCT/US2018/028189 (International Publication No. WO 2018/195215). Any combination or sub-combination of the features disclosed by the present application can be combined with any combination or sub-combination of the features disclosed by Patent Cooperation Treaty International Application No. PCT/US2018/028189 (International Publication No. WO 2018/195215).

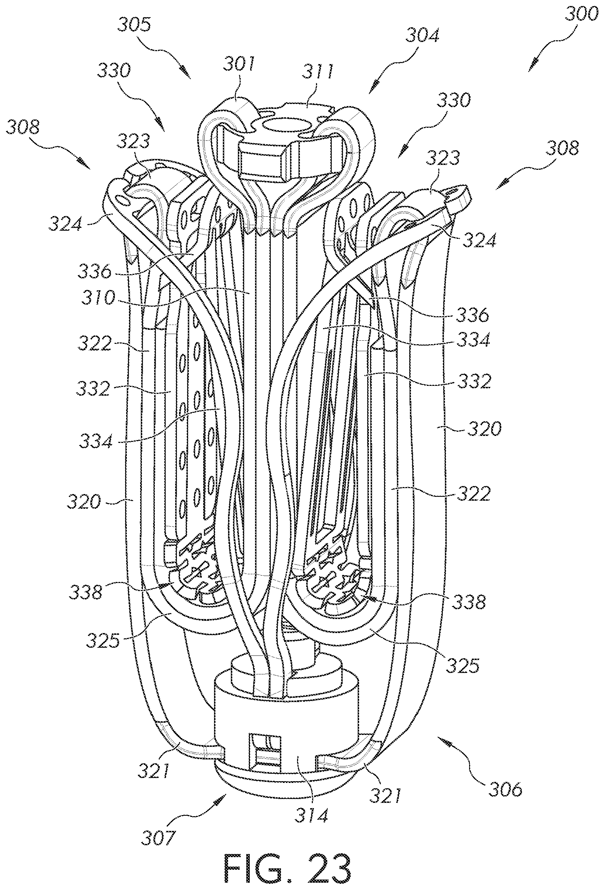

Referring now to FIG. 23, an example of an implantable device or implant 300 is shown. The implantable device 300 is one of the many different configurations that the device 100 that is schematically illustrated in FIGS. 8-14 can take. The device 300 can include any other features for an implantable device or implant discussed in the present application, and the device 300 can be positioned to engage valve tissue 20, 22 as part of any suitable valve repair system (e.g., any valve repair system disclosed in the present application).

The implantable device or implant 300 includes a proximal or attachment portion 305, an anchor portion 306, and a distal portion 307. In some implementations, the device/implant 300 includes a coaptation portion 304, and the coaptation portion 304 can optionally include a coaptation element 310 (e.g., spacer, plug, membrane, sheet, etc.) for implantation between the leaflets 20, 22 of the native valve. In some implementations, the anchor portion 306 includes a plurality of anchors 308. In some implementations, each anchor 308 can include one or more paddles, e.g., outer paddles 320, inner paddles 322, paddle extension members or paddle frames 324. The anchors can also include and/or be coupled to clasps 330. In some implementations, the attachment portion 305 includes a first or proximal collar 311 (or other attachment element) for engaging with a capture mechanism of a delivery system.

The anchors 308 can be attached to the other portions of the device and/or to each other in a variety of different ways (e.g., directly, indirectly, welding, sutures, adhesive, links, latches, integrally formed, a combination of some or all of these, etc.). In some implementations, the anchors 308 are attached to a coaptation element 310 by connection portions 325 and to a cap 314 by connection portions 321.

The anchors 308 can comprise first portions or outer paddles 320 and second portions or inner paddles 322 separated by connection portions 323. The connection portions 323 can be attached to paddle frames 324 that are hingeably attached to a cap 314 or other attachment portion. In this manner, the anchors 308 are configured similar to legs in that the inner paddles 322 are like upper portions of the legs, the outer paddles 320 are like lower portions of the legs, and the connection portions 323 are like knee portions of the legs.

In some implementations with a coaptation element 310, the coaptation element 310 and the anchors 308 can be coupled together in various ways. As shown in the illustrated example, the coaptation element 310 and the anchors 308 can be coupled together by integrally forming the coaptation element 310 and the anchors 308 as a single, unitary component. This can be accomplished, for example, by forming the coaptation element 310 and the anchors 308 from a continuous strip 301 of a braided or woven material, such as braided or woven nitinol wire. In the illustrated example, the coaptation element 310, the outer paddle portions 320, the inner paddle portions 322, and the connection portions 321, 323, 325 are formed from a continuous strip of fabric 301.

Like the anchors 208 of the implantable device or implant 200 described above, the anchors 308 can be configured to move between various configurations by axially moving the distal end of the device (e.g., cap 314, etc.) relative to the proximal end of the device (e.g., proximal collar 311 or other attachment element, etc.). This movement can be along a longitudinal axis extending between the distal end (e.g., cap 314, etc.) and the proximal end (e.g., collar 311 or other attachment element, etc.) of the device.

In some implementations, in the straight configuration, the paddle portions 320, 322 are aligned or straight in the direction of the longitudinal axis of the device. In some implementations, the connection portions 323 of the anchors 308 are adjacent the longitudinal axis of the spacer or coaptation element 310. From the straight configuration, the anchors 308 can be moved to a fully folded configuration (e.g., FIG. 23), e.g., by moving the proximal end and distal end toward each other and/or toward a midpoint or center of the device.

In some implementations, the clasps comprise a moveable arm coupled to an anchor. In some implementations, the clasps 330 include a base or fixed arm 332, a moveable arm 334, optional barbs/friction-enhancing elements 336, and a joint portion 338. The fixed arms 332 are attached to the inner paddles 322, with the joint portion 338 disposed proximate the coaptation element 310. The joint portion 338 is spring-loaded so that the fixed and moveable arms 332, 334 are biased toward each other when the clasp 330 is in a closed condition.

The fixed arms 332 are attached to the inner paddles 322 through holes or slots with sutures. The fixed arms 332 can be attached to the inner paddles 322 with any suitable means, such as screws or other fasteners, crimped sleeves, mechanical latches or snaps, welding, adhesive, or the like. The fixed arms 332 remain substantially stationary relative to the inner paddles 322 when the moveable arms 334 are opened to open the clasps 330 and expose the optional barbs 336. The clasps 330 are opened by applying tension to actuation lines attached to the moveable arms 334, thereby causing the moveable arms 334 to articulate, pivot, and/or flex on the joint portions 338.

In short, the implantable device or implant 300 is similar in configuration and operation to the implantable device or implant 200 described above, except that the coaptation element 310, outer paddles 320, inner paddles 322, and connection portions 321, 323, 325 are formed from the single strip of material 301. In some implementations, the strip of material 301 is attached to the proximal collar 311, cap 314, and paddle frames 324 by being woven or inserted through openings in the proximal collar 311, cap 314, and paddle frames 324 that are configured to receive the continuous strip of material 301. The continuous strip 301 can be a single layer of material or can include two or more layers. In some implementations, portions of the device 300 have a single layer of the strip of material 301 and other portions are formed from multiple overlapping or overlying layers of the strip of material 301.

For example, FIG. 23 shows a coaptation element 310 and inner paddles 322 formed from multiple overlapping layers of the strip of material 301. The single continuous strip of material 301 can start and end in various locations of the device 300. The ends of the strip of material 301 can be in the same location or different locations of the device 300. For example, in the illustrated example of FIG. 23, the strip of material 301 begins and ends in the location of the inner paddles 322.

As with the implantable device or implant 200 described above, the size of the coaptation element 310 can be selected to minimize the number of implants that a single patient will require (preferably one), while at the same time maintaining low transvalvular gradients. In particular, forming many components of the device 300 from the strip of material 301 allows the device 300 to be made smaller than the device 200. For example, in some implementations, the anterior-posterior distance at the top of the coaptation element 310 is less than 2 mm, and the medial-lateral distance of the device 300 (i.e., the width of the paddle frames 324 which are wider than the coaptation element 310) at its widest is about 5 mm.

Additional features of the device 300, modified versions of the device, delivery systems for the device, and methods for using the device and delivery system are disclosed by Patent Cooperation Treaty International Application No. PCT/US2019/055320 (International Publication No. WO 2020/076898). Any combination or sub-combination of the features disclosed by the present application can be combined with any combination or sub-combination of the features disclosed by Patent Cooperation Treaty International Application No. PCT/US2019/055320 (International Publication No. WO 2020/076898).

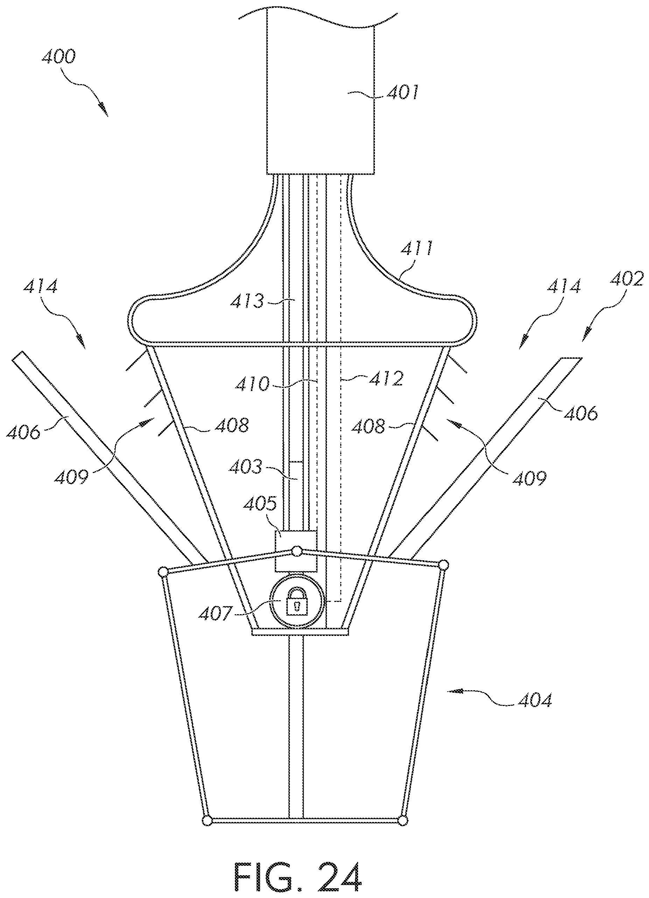

FIG. 24 illustrates another example of one of the many valve repair systems 400 for repairing a native valve of a patient that the concepts of the present application can be applied to. The valve repair system 400 includes a delivery device 401 and a valve repair device 402.

The valve repair device 402 includes a base assembly 404, a pair of paddles 406, and a pair of gripping members 408. In one example, the paddles 406 can be integrally formed with the base assembly. For example, the paddles 406 can be formed as extensions of links of the base assembly. In the illustrated example, the base assembly 404 of the valve repair device 402 has a shaft 403, a coupler 405 configured to move along the shaft, and a lock 407 configured to lock the coupler in a stationary position on the shaft. The coupler 405 is mechanically connected to the paddles 406, such that movement of the coupler 405 along the shaft 403 causes the paddles to move between an open position and a closed position. In this way, the coupler 405 serves as a means for mechanically coupling the paddles 406 to the shaft 403 and, when moving along the shaft 403, for causing the paddles 406 to move between their open and closed positions.

In some implementations, the gripping members 408 are pivotally connected to the base assembly 404 (e.g., the gripping members 408 can be pivotally connected to the shaft 403, or any other suitable member of the base assembly), such that the gripping members can be moved to adjust the width of the opening 414 between the paddles 406 and the gripping members 408. The gripping member 408 can optionally include a barbed portion 409 for attaching the gripping members to valve tissue when the valve repair device 402 is attached to the valve tissue. When the paddles 406 are in the closed position, the paddles engage the gripping members 408, such that, when valve tissue is attached to the barbed portion 409 of the gripping members, the paddles secure the valve repair device 402 to the valve tissue. In some implementations, the gripping members 408 are configured to engage the paddles 406 such that the barbed portion 409 engages the valve tissue member and the paddles 406 to secure the valve repair device 402 to the valve tissue member. For example, in certain situations, it can be advantageous to have the paddles 406 maintain an open position and have the gripping members 408 move outward toward the paddles 406 to engage valve tissue and the paddles 406.

While the example shown in FIG. 24 illustrates a pair of paddles 406 and a pair of gripping members 408, it should be understood that the valve repair device 402 can include any suitable number of paddles and gripping members.

In some implementations, the valve repair system 400 includes a placement shaft 413 that is removably attached to the shaft 403 of the base assembly 404 of the valve repair device 402. After the valve repair device 402 is secured to valve tissue, the placement shaft 413 is removed from the shaft 403 to remove the valve repair device 402 from the remainder of the valve repair system 400, such that the valve repair device 402 can remain attached to the valve tissue, and the delivery device 401 can be removed from a patient's body.

The valve repair system 400 can also include a paddle control mechanism 410, a gripper control mechanism 411, and a lock control mechanism 412. The paddle control mechanism 410 is mechanically attached to the coupler 405 to move the coupler along the shaft, which causes the paddles 406 to move between the open and closed positions. The paddle control mechanism 410 can take any suitable form, such as, for example, a shaft or rod. For example, the paddle control mechanism can comprise a hollow shaft, a catheter tube or a sleeve that fits over the placement shaft 413 and the shaft 403 and is connected to the coupler 405.

The gripper control mechanism 411 is configured to move the gripping members 408 such that the width of the opening 414 between the gripping members and the paddles 406 can be altered. The gripper control mechanism 411 can take any suitable form, such as, for example, a line, a suture or wire, a rod, a catheter, etc.

The lock control mechanism 412 is configured to lock and unlock the lock. The lock 407 locks the coupler 405 in a stationary position with respect to the shaft 403 and can take a wide variety of different forms and the type of lock control mechanism 412 may be dictated by the type of lock used. In examples in which the lock 407 includes a pivotable plate, the lock control mechanism 412 is configured to engage the pivotable plate to move the plate between the tilted and substantially non-tilted positions. The lock control mechanism 412 can be, for example, a rod, a suture, a wire, or any other member that is capable of moving a pivotable plate of the lock 407 between a tilted and substantially non-tilted position.

The valve repair device 402 is movable from an open position to a closed position. The base assembly 404 includes links that are moved by the coupler 405. The coupler 405 is movably attached to the shaft 403. In order to move the valve repair device from the open position to the closed position, the coupler 405 is moved along the shaft 403, which moves the links.

The gripper control mechanism 411 is moves the gripping members 408 to provide a wider or a narrower gap at the opening 414 between the gripping members and the paddles 406. In the illustrated example, the gripper control mechanism 411 includes a line, such as a suture, a wire, etc. that is connected to an opening in an end of the gripping members 408. When the line(s) is pulled, the gripping members 408 move inward, which causes the opening 414 between the gripping members and the paddles 406 to become wider.

In order to move the valve repair device 402 from the open position to the closed position, the lock 407 is moved to an unlocked condition by the lock control mechanism 412. Once the lock 407 is in the unlocked condition, the coupler 405 can be moved along the shaft 403 by the paddle control mechanism 410.

After the paddles 406 are moved to the closed position, the lock 407 is moved to the locked condition by the lock control mechanism 412 to maintain the valve repair device 402 in the closed position. After the valve repair device 402 is maintained in the locked condition by the lock 407, the valve repair device 402 is removed from the delivery device 401 by disconnecting the shaft 403 from the placement shaft 413. In addition, the valve repair device 402 is disengaged from the paddle control mechanism 410, the gripper control mechanism 411, and the lock control mechanism 412.

Additional features of the device 402, modified versions of the device, delivery systems for the device, and methods for using the device and delivery system are disclosed by Patent Cooperation Treaty International Application No. PCT/US2019/012707 (International Publication No. WO 2019139904). Any combination or sub-combination of the features disclosed by the present application can be combined with any combination or sub-combination of the features disclosed by Patent Cooperation Treaty International Application No. PCT/US2019/012707 (International Publication No. WO 2019139904).

Clasps or leaflet gripping devices disclosed herein can take a wide variety of different forms. Examples of clasps are disclosed by Patent Cooperation Treaty International Application No. PCT/US2018/028171 (International Publication No. WO 2018195201). Any combination or sub-combination of the features disclosed by the present application can be combined with any combination or sub-combination of the features disclosed by Patent Cooperation Treaty International Application No. PCT/US2018/028171 (International Publication No. WO 2018195201).

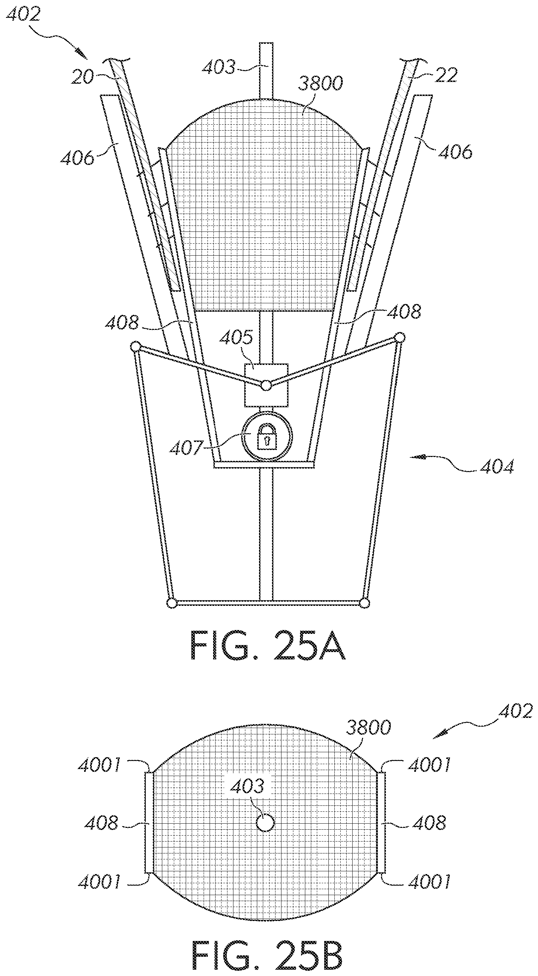

Referring to FIGS. 25A-25B, in some implementations the valve repair device 402 has a coaptation element 3800. The valve repair device 402 can have the same configuration as the valve repair device illustrated by FIG. 24 with the addition of the coaptation element. The coaptation element 3800 can take a wide variety of different forms. The coaptation element 3800 can be compressible and/or expandable. For example, the coaptation element can be compressed to fit inside one or more catheters of a delivery system, can expand when moved out of the one or more catheters, and/or can be compressed by the paddles 406 to adjust the size of the coaptation element. In the example illustrated by FIGS. 25A and 25B, the size of the coaptation element 3800 can be reduced by squeezing the coaptation element with the paddles 406 and can be increased by moving the paddles 406 away from one another. The coaptation element 3800 can extend past outer edges 4001 of the gripping members or clasps 408 as illustrated for providing additional surface area for closing the gap of a mitral valve.

The coaptation element 3800 can be coupled to the valve repair device 402 in a variety of different ways. For example, the coaptation element 3800 can be fixed to the shaft 403, can be slidably disposed around the shaft, can be connected to the coupler 405, can be connected to the lock 407, and/or can be connected to a central portion of the clasps or gripping members 408. In some implementations, the coupler 405 can take the form of the coaptation element 3800. That is, a single element can be used as the coupler 405 that causes the paddles 406 to move between the open and closed positions and the coaptation element 3800 that closes the gap between the leaflets 20, 22 when the valve repair device 402 is attached to the leaflets.

The coaptation element 3800 can be disposed around one or more of the shafts or other control elements of the valve repair system 400. For example, the coaptation element 3800 can be disposed around the shaft 403, the shaft 413, the paddle control mechanism 410, and/or the lock control mechanism 412.

The valve repair device 402 can include any other features for a valve repair device discussed in the present application, and the valve repair device 402 can be positioned to engage valve tissue as part of any suitable valve repair system (e.g., any valve repair system disclosed in the present application). Additional features of the device 402, modified versions of the device, delivery systems for the device, and methods for using the device and delivery system are disclosed by Patent Cooperation Treaty International Application No. PCT/US2019/012707 (International Publication No. WO 2019139904). Any combination or sub-combination of the features disclosed by the present application can be combined with any combination or sub-combination of the features disclosed by Patent Cooperation Treaty International Application No. PCT/US2019/012707 (International Publication No. WO 2019139904).

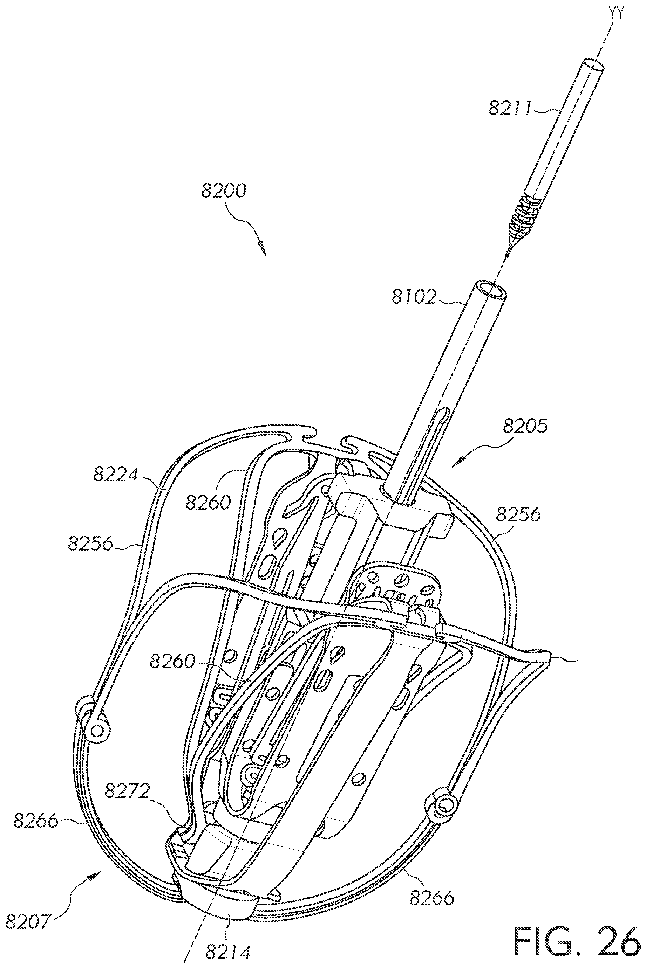

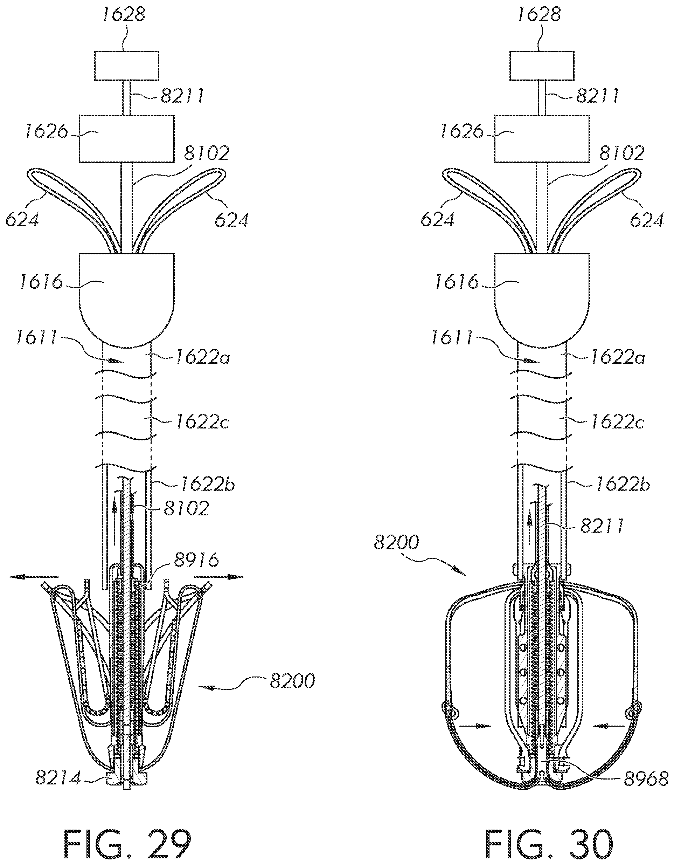

FIGS. 26-30 illustrate another example of one of the many valve repair systems for repairing a native valve of a patient that the concepts of the present application can be applied to. Referring to FIGS. 29 and 30, the valve repair system includes an implant catheter assembly 1611 and an implantable valve repair device 8200. Referring to FIGS. 26-28, the implantable device 8200 includes a proximal or attachment portion 8205, paddle frames 8224, and a distal portion 8207. The attachment portion 8205, the distal portion 8207, and the paddle frames 8224 can be configured in a variety of ways.

In the example illustrated in FIG. 26, the paddle frames 8224 can be symmetric along longitudinal axis YY. However, in some implementations, the paddle frames 8224 are not symmetric about the axis YY. Moreover, referring to FIG. 26, the paddle frames 8224 include outer frame portions 8256 and inner frame portions 8260.

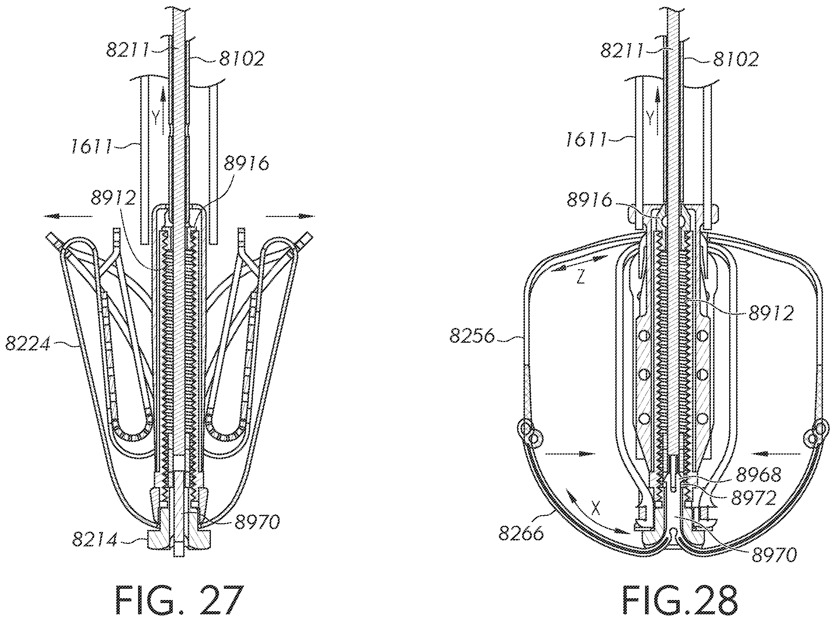

In some implementations, the connector 8266 (e.g., shaped metal component, shaped plastic component, tether, wire, strut, line, cord, suture, etc.) attaches to the outer frame portions 8256 at outer ends of the connector 8266 and to a coupler 8972 at an inner end 8968 of the connector 8266 (see FIG. 28). Between the connector 8266 and the attachment portion 8205, the outer frame portions 8256 form a curved shape. For example, in the illustrated example, the shape of the outer frame portions 8256 resembles an apple shape in which the outer frame portions 8256 are wider toward the attachment portion 8205 and narrower toward the distal portion 8207. In some implementations, however, the outer frame portions 8256 can be otherwise shaped.

The inner frame portions 8260 extend from the attachment portion 8205 toward the distal portion 8207. The inner frame portions 8260 then extend inward to form retaining portions 8272 that are attached to the actuation cap 8214. The retaining portions 8272 and the actuation cap 8214 can be configured to attach in any suitable manner.

In some implementations, the inner frame portions 8260 are rigid frame portions, while the outer frame portions 8256 are flexible frame portions. The proximal end of the outer frame portions 8256 connect to the proximal end of the inner frame portions 8260, as illustrated in FIG. 26.

The width adjustment element 8211 (e.g., width adjustment wire, width adjustment shaft, width adjustment tube, width adjustment line, width adjustment cord, width adjustment suture, width adjustment screw or bolt, etc.) is configured to move the outer frame portions 8256 from the expanded position to the narrowed position by pulling the inner end 8968 (FIG. 28) and portions of the connector 8266 into the actuation cap 8214. The actuation element 8102 is configured to move the inner frame portions 8260 to open and close the paddles in accordance with some implementations disclosed herein.

As shown in FIGS. 27 and 28, the connector 8266 has an inner end 8968 that engages with the width adjustment element 8211 such that a user can move the inner end 8968 inside the receiver 8912 (e.g., an internally threaded element, a column, a conduit, a hollow member, a notched receiving portion, a tube, a shaft, a sleeve, a post, a housing, a cylinder, tracks, etc.) to move the outer frame portions 8256 between a narrowed position and an expanded position. In the illustrated example, the inner end 8968 includes a post 8970 that attaches to the outer frame portions 8256 and a coupler 8972 that extends from the post 8970. The coupler 8972 is configured to attach and detach from both the width adjustment element 8211 and the receiver 8912. The coupler 8972 can take a wide variety of different forms. For example, the coupler 8972 can include one or more of a threaded connection, features that mate with threads, detent connections, such as outwardly biased arms, walls, or other portions. When the coupler 8972 is attached to the width adjustment element 8211, the coupler is released from the receiver 8912. When the coupler 8972 is detached from the width adjustment element 8211, the coupler is secured to the receiver. The inner end 8968 of the connector can, however, be configured in a variety of ways. Any configuration that can suitably attach the outer frame portions 8256 to the coupler to allow the width adjustment element 8211 to move the outer frame portions 8256 between the narrowed position and the expanded position can be used. The coupler can be configured in a variety of ways as well and can be a separate component or be integral with another portion of the device, e.g., of the connector or inner end of the connector.

The width adjustment element 8211 allows a user to expand or contract the outer frame portions 8256 of the implantable device 8200. In the example illustrated in FIGS. 27 and 28, the width adjustment element 8211 includes an externally threaded end that is threaded into the coupler 8972. The width adjustment element 8211 moves the coupler in the receiver 8912 to adjust the width of the outer frame portions 8256. When the width adjustment element 8211 is unscrewed from the coupler 8972, the coupler engages the inner surface of the receiver 8912 to set the width of the outer frame portions 8256.

In some implementations, the receiver 8912 can be integrally formed with a distal cap 8214. Moving the cap 8214 relative to a body of the attachment portion 8205 opens and closes the paddles. In the illustrated example, the receiver 8912 slides inside the body of the attachment portion. When the coupler 8972 is detached from the width adjustment element 8211, the width of the outer frame portions 8256 is fixed while the actuation element 8102 moves the receiver 8912 and cap 8214 relative to a body of the attachment portion 8205. Movement of the cap can open and close the device in the same manner as any of the implementations disclosed above.

In the illustrated example, a driver head 8916 is disposed at a proximal end of the actuation element 8102. The driver head 8916 releasably couples the actuation element 8102 to the receiver 8912. In the illustrated example, the width adjustment element 8211 extends through the actuation element 8102. The actuation element is axially advanced in the direction opposite to direction Y to move the distal cap 8214. Movement of the distal cap 8214 relative to the attachment portion 8205 is effective to open and close the paddles, as indicated by the arrows in FIG. 27. That is, movement of the distal cap 8214 in the direction Y closes the device and movement of the distal cap in the direction opposite to direction Y opens the device.

Also illustrated in FIGS. 27 and 28, the width adjustment element 8211 extends through the actuation element 8102, the driver head 8916, and the receiver 8912 to engage the coupler 8972 attached to the inner end 8968. The movement of the outer frame portions 8256 to the narrowed position can allow the device or implant 8200 to maneuver more easily into position for implantation in the heart by reducing the contact and/or friction between the native structures of the heart-e.g., chordae-and the device 8200. The movement of the outer frame portions 8256 to the expanded position provides the anchor portion of the device or implant 8200 with a larger surface area to engage and capture leaflet(s) of a native heart valve.

Referring to FIGS. 29 and 30, an implementation of an implant catheter assembly 1611 in which clasp actuation lines 624 extend through a handle 1616, the actuation element 8102 is coupled to a paddle actuation control 1626, and the width adjustment element 8211 is coupled to a paddle width control 1628. A proximal end portion 1622a of the shaft or catheter of the implant catheter assembly 1611 can be coupled to the handle 1616, and a distal end portion 1622b of the shaft or catheter can be coupled to the implantable device 8200. The actuation element 8102 can extend distally from the paddle actuation control 1626, through the handle 1616, through the delivery shaft or catheter of the implant catheter assembly 1611, and through the proximal end of the device 8200, where it couples with the driver head 8916. The actuation element 8102 can be axially movable relative to the outer shaft of the implant catheter assembly 1611 and the handle 1616 to open and close the device.

The width adjustment element 8211 can extend distally from the paddle width control 1628, through the paddle actuation control 1626 and through the actuation element 8102 (and, consequently, through the handle 1616, the outer shaft of the implant catheter assembly 1611, and through the device 8200), where it couples with the movable coupler 8972. The width adjustment element 8211 can be axially movable relative to the actuation element 8102, the outer shaft of the implant catheter assembly 1611, and the handle 1616. The clasp actuation lines 624 can extend through and be axially movable relative to the handle 1616 and the outer shaft of the implant catheter assembly 1611. The clasp actuation lines 624 can also be axially movable relative to the actuation element 8102.

Referring to FIGS. 29 and 30, the width adjustment element 8211 can be releasably coupled to the coupler 8972 of the device 8200. Advancing and retracting the width adjustment element 8211 with the paddle width control 1628 widens and narrows the paddles. Advancing and retracting the actuation element 8102 with the paddle actuation control 1626 opens and closes the paddles of the device.

In the examples of FIGS. 29 and 30, the catheter or shaft of the implant catheter assembly 1611 is an elongate shaft extending axially between the proximal end portion 1622a, which is coupled to the handle 1616, and the distal end portion 1622b, which is coupled to the device 8200. The outer shaft of the implant catheter assembly 1611 can also include an intermediate portion 1622c disposed between the proximal and distal end portions 1622a, 1622b.

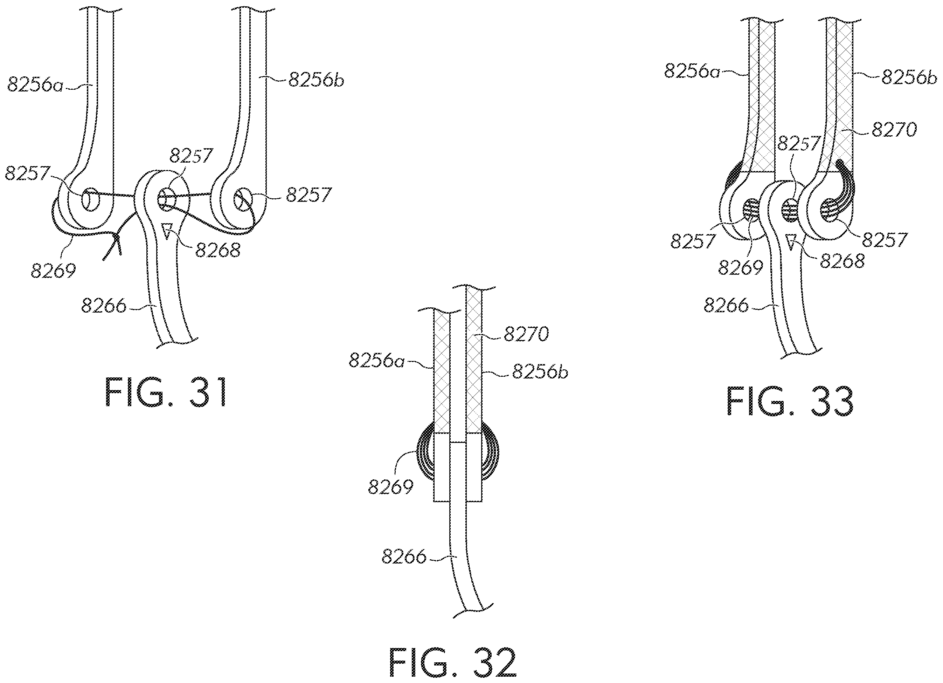

Turning now to FIGS. 31-35C, example interfaces between the outer frame portions 8256 and a connector 8266 of the device 8200 are shown in greater detail. Other interfaces are also possible. As illustrated in FIG. 31, the interface can include a first outer frame portion 8256a, a second outer frame portion 8256b, and a connector 8266. The connector 8266 is positioned between the first outer frame portion 8256a and the second outer frame portion 8256b, as shown in FIG. 32.

In some implementations, each of the first outer frame portion 8256a, the second outer frame portion 8256b, and the connector 8266 includes an eyelet 8257. The eyelets 8257 enable the first outer frame portion 8256a, the second outer frame portion 8256b, and the connector 8266 to be secured together, for example, through one or more sutures 8269. It should be appreciated, however, that the outer frame portions and the connection portions can be secured together through other securing means in some implementations. In some implementations, the securing means allow the eyelets 8257 to coaxially rotate in relationship with each other while remaining concentric during the widening/narrowing of the paddles. In some implementations, eyelets are not used, and other connection means are used.

As shown in FIG. 31, the connector 8266 further includes an optional connecting anchor 8268. The connecting anchor 8268 can take a variety of different forms. In the illustrated example, the connecting anchor 8268 is an additional opening or eyelet. The connecting anchor can be configured to stop or inhibit sliding movement of the suture 8269 therethrough. In some implementations, the connecting anchor 8268 provides an additional attachment point between the connector 8266 and the outer frame portions 8256a, 8256b, which can improve the strength of the interface.

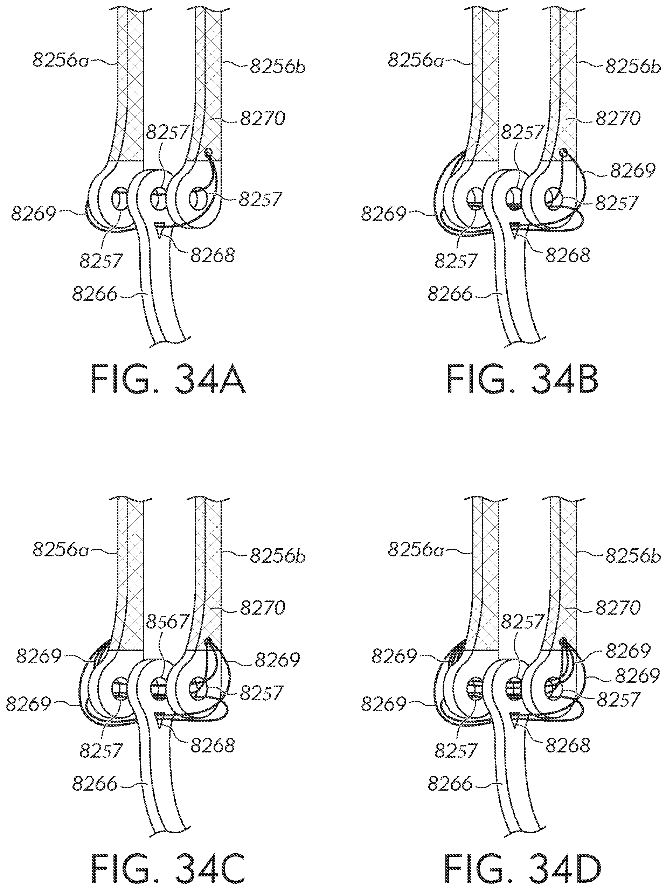

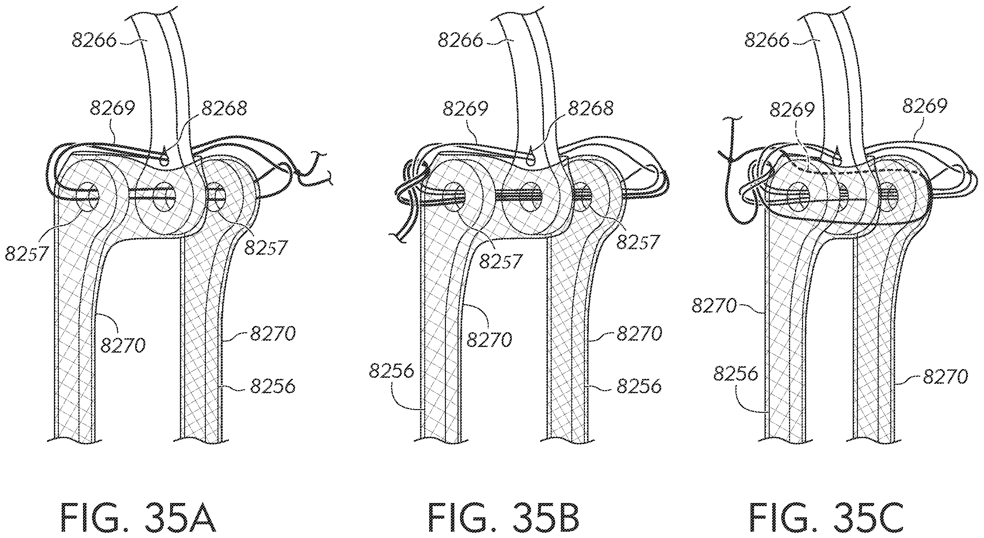

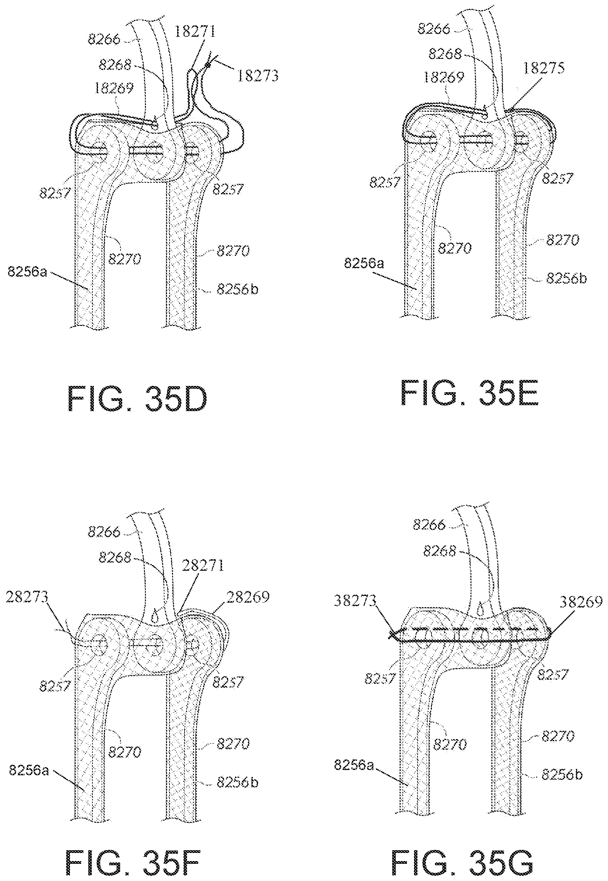

In the examples illustrated in FIGS. 32-34D, a sleeve 8270 is positioned over a length of the first and second outer frame portions 8256a, 8256b. The sleeve 8270 can take a variety of different forms and, in some implementations, is a cloth material, tubing, etc. and can be made from a wide variety of different materials, including but not limited to, plastic (e.g., polyethylene terephthalate (PET)), textiles, etc. However, some implementations do not include the sleeve. In some implementations, such as the implementations illustrated in FIGS. 35A-35C and 35D-35I, the sleeve 8270 can be positioned over the eyelets 8257 of the outer frame portion and/or the connector 8266. Accordingly, the sleeve 8270 is optional. When included the sleeve 8270 can provide a surface to which one or more of the sutures 8269 can be secured (as shown in FIGS. 32 and 33), which can reduce or prevent the suture from moving in place (e.g., sliding with respect to the eyelets 8257).

The sutures 8269 can be threaded through the eyelets 8257 and/or the connecting anchor 8268 in any one of a variety of paths. In the implementation illustrated in FIG. 31, the suture 8269 extends through the eyelet 8257 of the first outer frame portion 8256a, over the top of the first outer frame portion 8256a, through the eyelet 8257 of the connector 8266, over the top of the second outer frame portion 8256b, through the eyelet of the second outer frame portion 8256b, and through the eyelet 8257 of the connector 8266. In some implementations, the suture 8269 extends through the eyelet 8257 of the first outer frame portion 8256a, over the top of the first outer frame portion 8256a, through the eyelet 8257 of the connector 8266, through the eyelet of the second outer frame portion 8256b, over the top of the second outer frame portion 8256b, and through the eyelet 8257 of the connector 8266. It should be appreciated that although one suture 8269 is depicted in FIG. 31, additional sutures can be added to strengthen the interface.

In some implementations, such as the example illustrated in FIGS. 32 and 33, multiple sutures 8269 can be secured (e.g., stitched) to the sleeve 8270 of each of the first and second outer frame portions 8256a, 8256b and threaded through the eyelets 8257. Each suture can pass through the eyelets 8257 of the first and second outer frame portions 8256a, 8256b and the eyelet of the connector 8266 more than once. For example, a first suture extends through the eyelet 8257 of the first outer frame portion 8256a, the eyelet 8257 of the connector 8266, and the eyelet 8257 of the second outer frame portion 8256b, through the sleeve 8270 of the second outer frame portion 8256b, back through the eyelet 8257 of the second outer frame portion 8256b, the eyelet 8257 of the connector 8266, and the eyelet 8257 of the first outer frame portion 8256a, and through the sleeve 8270 of the first outer frame portion 8256a, before being joined at the ends of the suture 8269. A second suture 8269 extends through the eyelet 8257 of the second outer frame portion 8256b, the eyelet 8257 of the connector 8266, and the eyelet 8257 of the first outer frame portion 8256a, through the sleeve 8270 of the first outer frame portion 8256a, back through the eyelet 8257 of the first outer frame portion 8256a, the eyelet 8257 of the connector 8266, and the eyelet 8257 of the second outer frame portion 8256b, and through the sleeve 8270 of the second outer frame portion 8256b, before being joined at the ends of the suture 8269. Additional sutures 8269 can be included in some implementations. Moreover, in some implementations, the suture can be secured at each end to, for example, the sleeves 8270, instead of being looped and secured to itself at the ends.

The implementations illustrated in FIGS. 31-33 can be used to secure the connector 8266 to the outer frame portions 8256 to prevent or inhibit loosening and thereby enabling the connector 8266 and the outer frame portions 8256 to move such that the eyelets 8257 are not coaxially and concentrically aligned with one another, as shown in FIG. 28. In some implementations, one or more additional sutures 8269 can be passed through the connecting anchor 8268 to add strength to the interface. Additionally or alternatively, more than two sutures can be used to distribute the tensile stress throughout the interface.

FIGS. 34A-34D illustrate an implementation for securing the outer frame portions to the connector using four sutures 8269 and the connecting anchor 8268. For clarity, FIGS. 34A-34D illustrate each of the four sutures 8269 in a particular order, although the order of inserting the sutures is not limited. In FIG. 34A, a first suture 8269 extends through the sleeve 8270 of the second outer frame portion 8256b, the eyelet 8257 of the second outer frame portion 8256b, the eyelet 8257 of the connector 8266, and the eyelet 8257 of the first outer frame portion 8256a, before looping around the first outer frame portion 8256a, extending through the connecting anchor 8268, looping around the second outer frame portion 8256b and being joined at the ends of the suture 8269.

Next, in FIG. 34B, a second suture 8269 extends through the sleeve 8270 of the first outer frame portion 8256a, the eyelet 8257 of the first outer frame portion 8256a, the eyelet 8257 of the connector 8266, and the eyelet 8257 of the second outer frame portion 8256b, before looping around the second outer frame portion 8256b, extending through the connecting anchor 8268, looping around the first outer frame portion 8256a and being joined at the ends of the suture 8269.

In FIG. 34C, a third suture 8269 extends through the sleeve 8270 of the second outer frame portion 8256b, through the eyelets of the second outer frame portion 8256b, the connector 8266, and the first outer frame portion 8256a. The suture 8269 then extends through the sleeve 8270 of the first outer frame portion 8256a, back through the eyelets of the first outer frame portion 8256a, the connector 8266, and the second outer frame portion 8256b, and is joined to the other end of the suture 8269.

In FIG. 34D, a fourth suture 8269 extends through the sleeve 8270 of the first outer frame portion 8256a, through the eyelets of the first outer frame portion 8256a, the connector 8266, and the second outer frame portion 8256b. The suture 8269 then extends through the sleeve 8270 of the second outer frame portion 8256b, back through the eyelets of the second outer frame portion 8256b, the connector 8266, and the first outer frame portion 8256a, and is joined to the other end of the suture 8269.

Although the example in FIG. 34D includes four sutures, it is contemplated that one or more additional sutures can be added to further increase the strength of the interface. Moreover, in some implementations one or more of the additional sutures can be passed through the connecting anchor 8268. For example, in some implementations a first plurality of sutures can pass through the eyelets of the connector and the outer frame portions and not through the connecting anchor, while a second plurality of sutures can pass through the eyelets of the connector and the outer frame portions and through the connecting anchor. In some implementations, the sleeve 8270 on the outer frame portions can be omitted.

In FIGS. 35A-35C, a first sleeve 8270 covers the eyelet 8257 of one of the outer frame portions 8256 and the eyelet 8257 of the connector 8266, while a second sleeve 8270 covers the eyelet 8257 of the other outer frame portion 8256. The first sleeve 8270 can optionally also cover the connection anchor 8268 of the connector 8266. The suture 8269 can be folded in half to create a looped portion (e.g., a first loop) opposite free ends of the suture. The suture is passed through the first and second sleeves 8270, the eyelets 8257 of the outer frame portions 8256 and the connector 8266, and through the connecting anchor 8268, and the free ends of the suture 8269 are tied around the looped portion of the suture 8269, as shown in FIG. 35A, thereby forming a second loop. Once knotted, the free ends of the suture 8269 are sent back through the sleeves and the eyelets 8257 of the outer frame portions 8256 and the connector 8266 and tied around the second loop, as shown in FIG. 35B.

In some implementations, after being tied around the second loop, one of the free ends of the suture 8269 is threaded through the sleeves 8270 around the eyelets 8257 and is knotted to the other free end of the suture 8269, as shown in FIG. 35C. Although some implementations can reduce a profile of the interface by securing the sleeves 8270 in close proximity to the surfaces of the outer frame portions, the threading of the suture 8269 can be optional depending on the implementation. Accordingly, it is contemplated that in some implementations, the suture 8269 can be knotted after it is tied around the second loop in FIG. 35B.

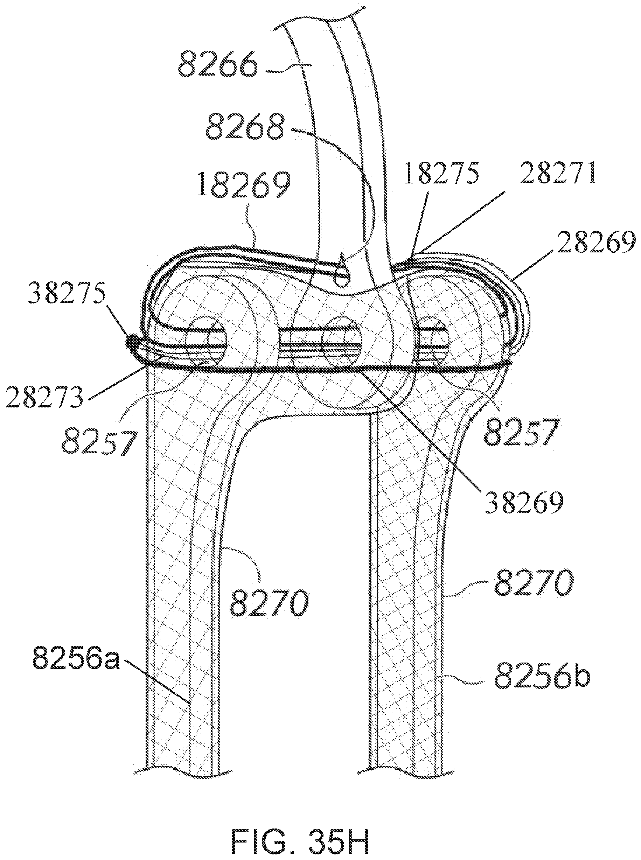

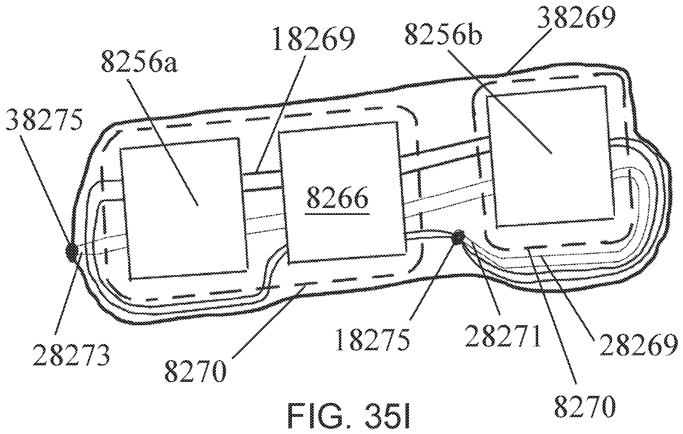

Referring to FIGS. 35D-35I, in some implementations three sutures 18269, 28269, 38269 are used to connect the outer frame portions 8256a, 8256b to the connector 8266. In FIGS. 35D-35I, the three sutures 18269, 28269, 38269 are illustrated by lines having different weights to allow for visual differentiation between the three sutures. However, the three sutures 18269, 28269, 38269 can be made from the same or different materials (e.g., the same or different sutures). In FIGS. 35E-35G, the three sutures 18269, 28269, 38269 are illustrated separately on the outer frame portions 8256a, 8256b and the connector 8266 to simplify the drawings, while FIGS. 35H and 35I illustrate all three sutures attaching the outer frame portions 8256a, 8256b to the connector 8266. The order of attaching the sutures is not limited.

In FIGS. 35D-35I, an optional first sleeve 8270 covers the eyelet 8257 of one of the outer frame portions 8256 and the eyelet 8257 of the connector 8266, while an optional second sleeve 8270 covers the eyelet 8257 of the other outer frame portion 8256. The first sleeve 8270 can optionally also cover the connection anchor 8268 of the connector 8266.

Referring to FIGS. 35D, 35E, 35H and 35I, the first suture 18269 can be folded in half to create a looped portion 18271 (e.g., a first loop) and opposite free ends 18273 of the first suture. The first suture 18269 is passed through the first and second sleeves 8270, the eyelets 8257 of the outer frame portions 8256 and the connector 8266, and through the connecting anchor 8268. The free ends 18273 of the suture 18269 are tied together with the looped portion 18271 of the suture 18269 to form a knot 18275. FIG. 35D shows first suture 18269 loosely positioned and FIG. 35E shows the first suture 18269 tightened and tied to form knot 18275.

Referring to FIGS. 35F, 35H and 35I, the second suture 28269 can be folded in half to create a looped portion 28271 (e.g., a first loop) opposite free ends 28273 of the second suture. The second suture 28269 is passed through the first and second sleeves 8270, the eyelets 8257 of the outer frame portions 8256 and the connector 8266. The free ends 28273 of the suture 28269 are positioned to a side of one of the outer frame portions 8256. The looped portion 28271 of the suture 18269 is tied together with the looped portion 18271 and free ends 18271 of the first suture 18269 at the knot 18275.

Referring to FIGS. 35G, 35H and 35I, the third suture 38269 is wrapped around the outer frame portions 8256 and the connector 8266. In the implementation illustrated by FIGS. 35G, 35H, and 35I, the third suture is wrapped around the outside of the first and second sleeves 8270. In some implementations, the third suture is stitched into the first and second sleeves 8270 one or more times. The free ends 38273 of the suture 38269 are positioned to a side of one of the outer frame portions 8256, with the free ends 28273 of the suture 28269. The free ends 28273 of the suture 28269 are tied together with the free ends 38273 of the suture 38269 to form a knot 38275. As such, the three sutures 18269, 28269, and 38269, are all tied together to connect the optional sleeves 8270 (when included), the outer frame portions 8256, and the connector 8266 together.

Although the example in FIGS. 35D includes three sutures, it is contemplated that one or more additional sutures can be added or one or more of the illustrated sutures can be omitted. For example, in some implementations a first plurality of sutures can pass through the eyelets of the connector and the outer frame portions and not through the connecting anchor, while a second plurality of sutures can pass through the eyelets of the connector and the outer frame portions and through the connecting anchor. In some implementations, the sleeves 8270 can be omitted.

In any of the examples set forth herein, it is contemplated that all or part of the suture can include a color or other visual indicator to enable the suture to be identified by a user. The visual indicator, for example, can enable the suture to be visually observed using x-ray or other imaging techniques that are utilized during delivery of the implantable device.

The interface between the outer frame portions and the connector of each paddle is not the only portion of the implantable device that can experience stresses during implant adjustment and delivery. The connector 8266 can also experience strain during paddle narrowing and, specifically, when the connector 8266 is pulled into the actuation cap 8214.

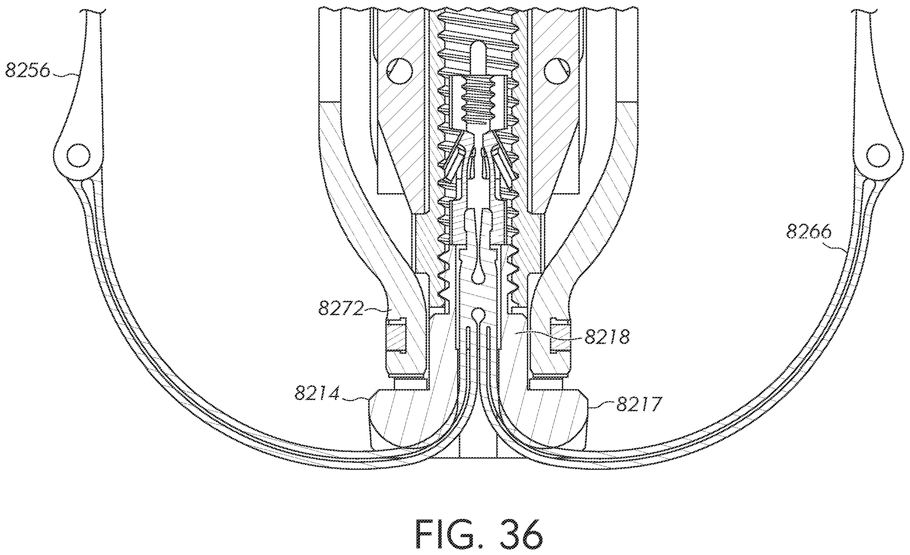

FIG. 36 illustrates a distal portion of an example implantable device including paddles having an adjustable width. As in various other examples described herein, the connector 8266 is coupled to an outer frame portion 8256 for each paddle. The actuation cap 8214 extends from a distal end of the retaining portion 8272. As shown in FIG. 36, the actuation cap 8214 includes a rounded portion 8217 and an attachment portion 8218. The rounded portion 8217 extends between the retaining portion 8272 and the connector 8266 while the attachment portion 8218 extends into the retaining portion 8272 and couples the actuation cap 8214 to the retaining portion 8272. The rounded portion 8217 of the actuation cap 8214 exhibits a force on the connector 8266, which can cause deformation or strain in the connector 8266. Accordingly, in some implementations, features can be incorporated to reduce the strain on the connector 8266.

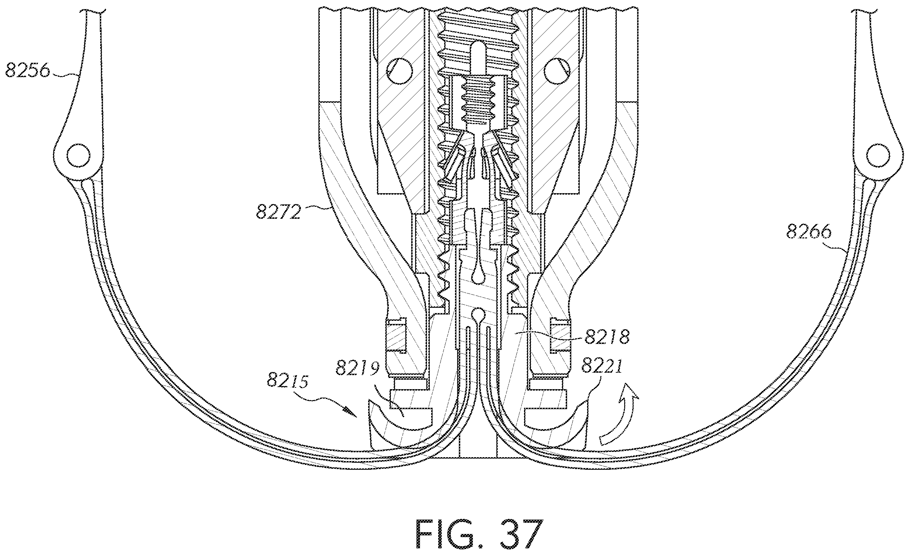

In some implementations, the actuation cap 8214 includes one or more flexible portions 8215. The one or more flexible portions can take a wide variety of different forms. In the example illustrated by FIG. 37 a flexible portion 8215 of the actuation cap 8214 is formed by an annular undercut 8219 that forms a thin, flexible cap end 8221, as shown in FIG. 37. The example flexible portion 8215 is positioned between the connector 8266 of the paddle frame and the retaining portion 8272 of the inner paddle frame portion. The flexible portion 8215 includes a radius of curvature, which can be similar to the radius of curvature of the rounded portion 8217 in FIG. 36. However, the flexible portion 8215 has greater flexibility in the as compared to the rounded portion 8217. Accordingly, the flexible portion 8215 of the actuation cap 8214 can flex in the direction of the arrow in FIG. 37 and toward the retaining portion 8272 as the connector 8266 is pulled into the retaining portion 8272 to narrow the paddles and/or as the actuation cap 8214 is moved relative to the body of the attachment portion 8205 to open and close the paddles, as described above. The flexure of the flexible portion 8215 can be effective to reduce the strain on the connector 8266.

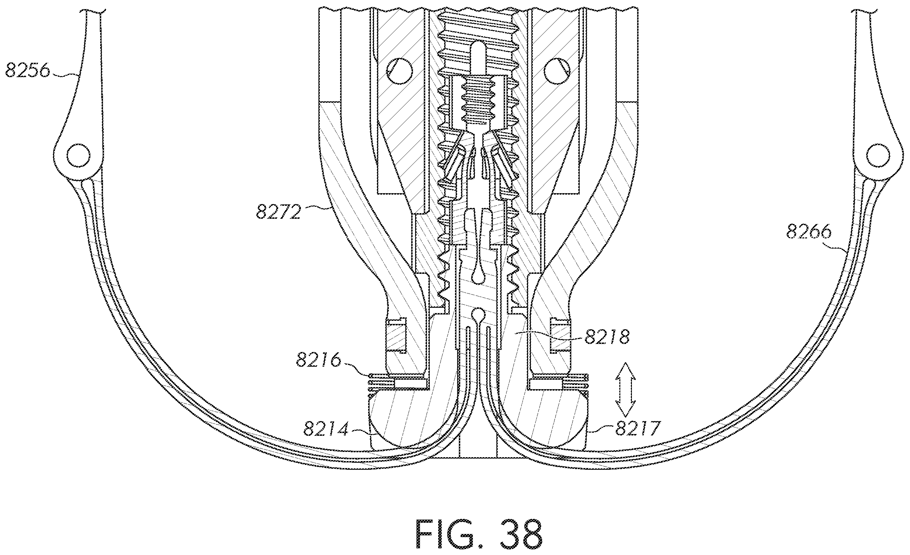

In some implementations, the actuation cap 8214 includes a rounded portion 8217 and an attachment portion 8218, as shown in FIG. 38. As in the example shown in FIG. 36, the rounded portion 8217 extends between the retaining portion 8272 and the connector 8266 while the attachment portion 8218 extends into the retaining portion 8272 and couples the actuation cap 8214 to the retaining portion 8272. However, in contrast to the example in FIG. 36, in FIG. 38, a biasing element 8216, such as the illustrated spring is positioned between the rounded portion 8217 and the retaining portion 8272. The biasing element 8216 can be, for example, a coil spring or a wave spring, although other types of biasing elements can be used depending on the implementation. In some implementations, a compressible material can be used as an alternative to the illustrated spring 8216. The biasing element 8216 can be made of metal or any other material known and used in the art.

In some implementations, the biasing element 8216 is configured to compress and/or expand in response to the forces between the rounded portion 8217 of the actuation cap 8214 and the retaining portion 8272, including but not limited to the forces generated on the rounded portion 8217 by the pulling of the connector 8266 into the retaining portion 8272. For example, as the connector 8266 is pulled into the retaining portion 8272 and against the rounded portion 8217 of the actuation cap 8214, the rounded portion 8217 of the actuation cap 8214 can be displaced toward the retaining portion 8272 by compression of the biasing element 8216, which reduces the overall load. Put another way, a portion of the force between the rounded portion 8217 and the connector 8266 can be transferred to the biasing element 8216, thereby compressing and/or expanding the biasing element 8216 and enabling the rounded portion 8217 to move in the direction of the arrows in FIG. 38. The reduction of the force, in turn, leads to a reduction of the strain on the connector 8266.

Various implementations described herein incorporate one or more features to adjust a distribution of force throughout the implantable device in an effort to reduce deformation of the implantable device over time, which can lead to improved function of the device during implant and/or an increased lifetime of the device. Other advantages can also be realized depending on the particular implementation.

The treatment techniques, methods, operations, steps, etc. described or suggested herein can be performed on a living subject (e.g., human, other animal, etc.) or on a non-living simulation, such as a cadaver, cadaver heart, simulator, imaginary person, etc.). When performed on a simulation, the body parts, e.g., heart, tissue, valve, etc., can optionally be referred to as "simulated" (e.g., simulated heart, simulated tissue, simulated valve, etc.) and can comprise, for example, computerized and/or physical representations of body parts, tissue, etc.

Any of the various systems, assemblies, devices, apparatuses, etc. in this disclosure can be sterilized (e.g., with heat, radiation, ethylene oxide, hydrogen peroxide, etc.) to ensure they are safe for use with patients, and the methods herein can comprise (or additional methods comprise or consist of) sterilization of the associated system, device, apparatus, etc. (e.g., with heat, radiation, ethylene oxide, hydrogen peroxide, etc.).

While various inventive aspects, concepts and features of the disclosures can be described and illustrated herein as embodied in combination in the examples herein, these various aspects, concepts, and features can be used in many alternative examples, either individually or in various combinations and sub-combinations thereof. Unless expressly excluded herein all such combinations and sub-combinations are intended to be within the scope of the present application. Still further, while various alternative examples as to the various aspects, concepts, and features of the disclosures-such as alternative materials, structures, configurations, methods, devices, and components, alternatives as to form, fit, and function, and so on-may be described herein, such descriptions are not intended to be a complete or exhaustive list of available alternative examples, whether presently known or later developed. Those skilled in the art can readily adopt one or more of the inventive aspects, concepts, or features into additional examples and uses within the scope of the present application even if such examples are not expressly disclosed herein.

Additionally, even though some features, concepts, or aspects of the disclosures may be described herein as being a preferred arrangement or method, such description is not intended to suggest that such feature is required or necessary unless expressly so stated. Still further, example or representative values and ranges may be included to assist in understanding the present application, however, such values and ranges are not to be construed in a limiting sense and are intended to be critical values or ranges only if so expressly stated.

Moreover, while various aspects, features and concepts may be expressly identified herein as being inventive or forming part of a disclosure, such identification is not intended to be exclusive, but rather there may be inventive aspects, concepts, and features that are fully described herein without being expressly identified as such or as part of a specific disclosure. Descriptions of example methods or processes are not limited to inclusion of all steps as being required in all cases, nor is the order that the steps are presented to be construed as required or necessary unless expressly so stated.

## Claims

1. An implantable device for repairing a native valve of a heart, the implantable device comprising:
an anchor portion (206) configured to connect to leaflets of the native valve;
wherein the anchor portion (206) includes a pair of frame portions (8256, 8256a, 8256b) that are movable between an expanded position and a narrowed position having a frame width that is less than the frame width when in the expanded position;
wherein each of the pair of frame portions (8256, 8256a, 8256b) have an eyelet (8257);
a connector (8266) having an eyelet (8257) and a connecting anchor (8268) proximate a first end of the connector (8266), the eyelet (8257) of the connector (8266) being positioned between the connecting anchor (8268) and the first end of the connector (8266), the connector (8266) at least partially positioned between the pair of frame portions (8256, 8256a, 8256b) such that the eyelets (8257) of the pair of frame portions (8256, 8256a, 8256b) and the eyelet (8257) of the connector (8266) are aligned; and
a plurality of sutures (8269) passing through the eyelet (8257) of the connector (8266) and the eyelet (8257) of each of the pair of frame portions (8256, 8256a, 8256b), wherein the plurality of sutures is configured to secure the pair of frame portions to the connector (8266).

2. The implantable device of claim 1, wherein at least one of the plurality of sutures (8269) passes through the connecting anchor (8268).

3. The implantable device of any preceding claim, wherein the plurality of sutures (8269) comprises at least two sutures passing through the connecting anchor (8268).

4. The implantable device of any preceding claim, wherein at least one of the plurality of sutures (8269) passes through the eyelet (8257) of the connector and the eyelet of each of the pair of frame portions (8256, 8256a, 8256b) more than once.

5. The implantable device of any preceding claim, wherein each of the plurality of sutures (8269) is secured to itself.

6. The implantable device of any preceding claim, wherein the plurality of sutures (8269) comprises:
a first suture (18269) and a second suture (28269) passing through the eyelet (8257) of the connector and the eyelet (8257) of each of the pair of frame portions (8256, 8256a, 8256b) and not passing through the connecting anchor (8268); and
a third suture (38269) and a fourth suture (8269) passing through the eyelet (8257) of the connector, the eyelet (8257) of each of the pair of frame portions (8256, 8256a, 8256b), and the connecting anchor (8268).

7. The implantable device of claim 6, further comprising a sleeve (8270) disposed about the pair of frame portions (8256, 8256a, 8256b).

8. The implantable device of claim 7, wherein:
each of the first suture (18269) and the second suture (28269) pass through the eyelet (8257) of the connector and the (8257) eyelet of each of the pair of frame portions (8256, 8256a, 8256b), through the sleeve (8270) of a first frame portion of the pair of frame portions (8256, 8256a, 8256b) and back through the eyelet of the connector and the eyelet (8257) of each of the pair of frame portions (8256, 8256a, 8256b).

9. The implantable device of any preceding claim, wherein the pair of frame portions (8256, 8256a, 8256b) coaxially rotate relative to the connector while the pair of frame portions (8256, 8256a, 8256b) move between the expanded position and the narrowed position.

10. The implantable device of any preceding claim, wherein the eyelets (8257) of the pair of frame portions (8256, 8256a, 8256b) and the eyelet (8257) of the connector are aligned along a common central axis while the pair of frame portions (8256, 8256a, 8256b) move between the expanded position and the narrowed position.

11. The implantable device of any preceding claim,
wherein the plurality of sutures (8269) passing through the eyelet (8257) of the connector and the eyelet (8257) of each of the pair of frame portions (8256, 8256a, 8256b) is a first plurality of sutures; the implantable device comprising:
a second plurality of sutures passing through the eyelet (8257) of the connector, the eyelet of each of the pair of frame portions (8256, 8256a, 8256b), and the connecting anchor (8268);
wherein the first plurality of sutures (8269) do not pass through the connecting anchor (8268); and
wherein the first plurality of sutures (8269) and the second plurality of sutures are configured to secure the connector between the pair of frame portions (8256, 8256a, 8256b) such that the pair of frame portions (8256, 8256a, 8256b) coaxially rotate relative to the connector while the pair of frame portions (8256, 8256a, 8256b) move between the expanded position and the narrowed position.

12. The implantable device of claim 11, wherein the first plurality of sutures passes through the eyelet (8257) of the connector and the eyelet (8257) of each of the pair of frame portions (8256, 8256a, 8256b) more than once.

13. The implantable device of claim 11 or claim 12, further comprising a sleeve (8270) disposed about the pair of frame portions (8256, 8256a, 8256b).

14. The implantable device of claim 13, wherein at least some of the first plurality of sutures (8269), the second plurality of sutures, or the first and second plurality of sutures pass through the sleeve (8270) of at least one of the pair of frame portions (8256, 8256a, 8256b).

15. The implantable device of any of claims 11-14, wherein each suture of the first plurality of sutures (8269) is secured to itself.

## Patentansprüche

1. Implantierbare Vorrichtung zur Reparatur einer nativen Herzklappe, wobei die implantierbare Vorrichtung umfasst:
einen Ankerabschnitt (206), der dazu ausgelegt ist, sich mit den Klappensegeln der nativen Herzklappe zu verbinden;
wobei der Ankerabschnitt (206) ein Paar von Rahmenabschnitten (8256, 8256a, 8256b) aufweist, die zwischen einer expandierten Position und einer verengten Position, die eine Rahmenbreite aufweist, die kleiner als die Rahmenbreite in der expandierten Position ist, beweglich sind;
wobei jeder des Paares von Rahmenabschnitten (8256, 8256a, 8256b) eine Öse (8257) aufweist;
einen Verbinder (8266) mit einer Öse (8257) und einem Verbindungsanker (8268) nahe einem ersten Ende des Verbinders (8266), wobei die Öse (8257) des Verbinders (8266) zwischen dem Verbindungsanker (8268) und dem ersten Ende des Verbinders (8266) positioniert ist, wobei der Verbinder (8266) zumindest teilweise zwischen dem Paar von Rahmenabschnitten (8256, 8256a, 8256b) positioniert ist, so dass die Ösen (8257) des Paares von Rahmenabschnitten (8256, 8256a, 8256b) und die Öse (8257) des Verbinders (8266) fluchten; und
eine Vielzahl von Nähten (8269), die durch die Öse (8257) des Verbinders (8266) und die Öse (8257) jedes des Paares von Rahmenabschnitten (8256, 8256a, 8256b) verlaufen, wobei die Vielzahl von Nähten dazu ausgelegt ist, die beiden Rahmenabschnitte am Verbinder (8266) zu befestigen.

2. Implantierbare Vorrichtung gemäß Anspruch 1, wobei zumindest eine der Vielzahl von Nähten (8269) durch den Verbindungsanker (8268) verläuft.

3. Implantierbare Vorrichtung gemäß einem der vorangehenden Ansprüche, wobei die Vielzahl von Nähten (8269) zumindest zwei Nähte umfasst, die durch den Verbindungsanker (8268) verlaufen.

4. Implantierbare Vorrichtung gemäß einem der vorangehenden Ansprüche, wobei zumindest eine der Vielzahl von Nähten (8269) mehr als einmal durch die Öse (8257) des Verbinders und die Öse jedes des Paares von Rahmenabschnitten (8256, 8256a, 8256b) verläuft.

5. Implantierbare Vorrichtung gemäß einem der vorangehenden Ansprüche, wobei jede der Vielzahl von Nähten (8269) an sich selbst befestigt ist.

6. Implantierbare Vorrichtung gemäß einem der vorangehenden Ansprüche, wobei die Vielzahl von Nähten (8269) umfasst:
eine erste Naht (18269) und eine zweite Naht (28269), die durch die Öse (8257) des Verbinders und die Öse (8257) jedes des Paares von Rahmenabschnitten (8256, 8256a, 8256b) und nicht durch den Verbindungsanker (8268) verlaufen; und
eine dritte Naht (38269) und eine vierte Naht (8269), die durch die Öse (8257) des Verbinders, die Öse (8257) jedes des Paares von Rahmenabschnitten (8256, 8256a, 8256b), und den Verbindungsanker (8268) verlaufen.

7. Implantierbare Vorrichtung gemäß Anspruch 6, ferner umfassend eine Hülse (8270), die um das Paar von Rahmenabschnitten (8256, 8256a, 8256b) angeordnet ist.

8. Implantierbare Vorrichtung gemäß Anspruch 7, wobei:
jede der ersten Naht (18269) und der zweiten Naht (28269) durch die Öse (8257) des Verbinders und durch die Öse (8257) jedes des Paares von Rahmenabschnitten (8256, 8256a, 8256b), durch die Hülse (8270) eines ersten Rahmenabschnittes des Paares von Rahmenabschnitten (8256, 8256a, 8256b) und zurück durch die Öse des Verbinders und die Öse (8257) jedes des Paares von Rahmenabschnitten (8256, 8256a, 8256b) verläuft.

9. Implantierbare Vorrichtung gemäß einem der vorangehenden Ansprüche, wobei sich das Paar von Rahmenabschnitten (8256, 8256a, 8256b) koaxial relativ zu dem Verbinder dreht, während sich das Paar von Rahmenabschnitten (8256, 8256a, 8256b) zwischen der expandierten Position und der verengten Position bewegt.

10. Implantierbare Vorrichtung gemäß einem der vorangehenden Ansprüche,
wobei die Ösen (8257) des Paares von Rahmenabschnitten (8256, 8256a, 8256b) und die Öse (8257) des Verbinders entlang einer gemeinsamen zentralen Achse ausgerichtet sind, während sich das Paar von Rahmenabschnitten (8256, 8256a, 8256b) zwischen der expandierten Position und der verengten Position bewegt.

11. Implantierbare Vorrichtung gemäß einem der vorangehenden Ansprüche,
wobei die Vielzahl von Fäden (8269), die durch die Öse (8257) des Verbinders und die Öse (8257) jedes des Paares von Rahmenabschnitten (8256, 8256a, 8256b) verlaufen, eine erste Vielzahl von Fäden darstellt; wobei die implantierbare Vorrichtung umfasst:
eine zweite Vielzahl von Fäden, die durch die Öse (8257) des Verbinders, die Öse jedes des Paares von Rahmenabschnitten (8256, 8256a, 8256b) und den Verbindungsanker (8268) verlaufen;
wobei die erste Vielzahl von Fäden (8269) nicht durch den Verbindungsanker (8268) verläuft; und
wobei die erste Vielzahl von Nähten (8269) und die zweite Vielzahl von Nähten dazu ausgelegt sind, den Verbinder zwischen dem Paar von Rahmenabschnitten (8256, 8256a, 8256b) zu sichern, so dass sich das Paar von Rahmenabschnitten (8256, 8256a, 8256b) koaxial relativ zum Verbinder dreht, während sich das Paar von Rahmenabschnitten (8256, 8256a, 8256b) zwischen der expandierten und der verengten Position bewegt.

12. Implantierbare Vorrichtung gemäß Anspruch 11,
wobei die erste Vielzahl von Nähten mehr als einmal durch die Öse (8257) des Verbinders und durch die Öse (8257) jedes des Paares von Rahmenabschnitten (8256, 8256a, 8256b) verlaufen.

13. Implantierbare Vorrichtung gemäß Anspruch 11 oder 12, ferner umfassend eine Hülse (8270) die um das Paar von Rahmenteilen (8256, 8256a, 8256b) angeordnet ist.

14. Implantierbare Vorrichtung gemäß Anspruch 13, wobei zumindest einige der ersten Vielzahl von Nähten (8269), die zweite Vielzahl von Nähten, oder die erste und die zweite Vielzahl von Nähten durch die Hülse (8270) des zumindest einen des Paares von Rahmenabschnitten (8256, 8256a, 8256b) verlaufen.

15. Implantierbare Vorrichtung gemäß einem der Ansprüche 11 - 14, wobei jede Naht der Vielzahl von Nähten (8269) an sich selbst befestigt ist.

## Revendications

1. Dispositif implantable destiné à réparer une valvule native d'un cœur, le dispositif implantable comprenant :
une partie d'ancrage (206) conçue pour se raccorder à des feuillets de la valvule native ;
la partie d'ancrage (206) comportant une paire de parties cadre (8256, 8256a, 8256b) qui sont mobiles entre une position déployée et une position rétrécie présentant une largeur de cadre qui est inférieure à la largeur de cadre dans la position déployée ;
chaque partie de la paire de parties cadre (8256, 8256a, 8256b) présentant un œillet (8257) ;
un raccord (8266) présentant un œillet (8257) et un ancrage de raccordement (8268) à proximité d'une première extrémité du raccord (8266), l'œillet (8257) du raccord (8266) étant positionné entre l'ancrage de raccordement (8268) et la première extrémité du raccord (8266), le raccord (8266) étant au moins partiellement positionné entre la paire de parties cadre (8256, 8256a, 8256b) de telle sorte que les œillets (8257) de la paire de parties cadre (8256, 8256a, 8256b) et l'œillet (8257) du raccord (8266) sont alignés ; et
une pluralité de sutures (8269) passant à travers l'œillet (8257) du raccord (8266) et l'œillet (8257) de chaque partie de la paire de parties cadre (8256, 8256a, 8256b), la pluralité de sutures étant conçues pour fixer de manière sûre la paire de parties cadre au raccord (8266).

2. Dispositif implantable selon la revendication 1, au moins une suture parmi la pluralité de sutures (8269) passant à travers l'ancrage de raccordement (8268).

3. Dispositif implantable selon l'une quelconque revendication précédente, la pluralité de sutures (8269) comprenant au moins deux sutures passant à travers l'ancrage de raccordement (8268).

4. Dispositif implantable selon l'une quelconque revendication précédente, au moins une suture parmi la pluralité de sutures (8269) passant plus d'une fois à travers l'œillet (8257) du raccord et l'œillet de chaque partie de la paire de parties cadre (8256, 8256a, 8256b).

5. Dispositif implantable selon l'une quelconque revendication précédente, chaque suture de la pluralité de sutures (8269) étant fixée de manière sûre à elle-même.

6. Dispositif implantable selon l'une quelconque revendication précédente, la pluralité de sutures (8269) comprenant :
une première suture (18269) et une deuxième suture (28269) passant à travers l'œil-let (8257) du raccord et l'œillet (8257) de chaque partie de la paire de parties cadre (8256, 8256a, 8256b) et ne passant pas à travers l'ancrage de raccordement (8268) ; et
une troisième suture (38269) et une quatrième suture (8269) passant à travers l'œil-let (8257) du raccord, l'œillet (8257) de chaque partie de la paire de parties cadre (8256, 8256a, 8256b) et l'ancrage de raccordement (8268).

7. Dispositif implantable selon la revendication 6, comprenant en outre un manchon (8270) disposé autour de la paire de parties cadre (8256, 8256a, 8256b).

8. Dispositif implantable selon la revendication 7 :
chaque suture de la première suture (18269) et de la deuxième suture (28269) passant à travers l'œillet (8257) du raccord et l'œillet (8257) de chaque partie de la paire de parties cadre (8256, 8256a, 8256b), à travers le manchon (8270) d'une première partie cadre de la paire de parties cadre (8256, 8256a, 8256b) et revenant à travers l'œillet du raccord et l'œillet (8257) de chaque partie de la paire de parties cadre (8256, 8256a, 8256b).

9. Dispositif implantable selon l'une quelconque revendication précédente, la paire de parties cadre (8256, 8256a, 8256b) tournant de manière coaxiale par rapport au raccord pendant que la paire de parties cadre (8256, 8256a, 8256b) se déplace entre la position déployée et la position rétrécie.

10. Dispositif implantable selon l'une quelconque revendication précédente, les œillets (8257) de la paire de parties cadre (8256, 8256a, 8256b) et l'œillet (8257) du raccord étant alignés le long d'un axe central commun pendant que la paire de parties cadre (8256, 8256a, 8256b) se déplace entre la position déployée et la position rétrécie.

11. Dispositif implantable selon l'une quelconque revendication précédente,
la pluralité de sutures (8269) passant à travers l'œillet (8257) du raccord et l'œillet (8257) de chaque partie de la paire de parties cadre (8256, 8256a, 8256b) étant une première pluralité de sutures ; le dispositif implantable comprenant :
une seconde pluralité de sutures passant à travers l'œillet (8257) du raccord, l'œillet de chaque partie de la paire de parties cadre (8256, 8256a, 8256b) et l'ancrage de raccordement (8268) ;
la première pluralité de sutures (8269) ne passant pas à travers l'ancrage de raccordement (8268) ; et
la première pluralité de sutures (8269) et la seconde pluralité de sutures étant conçues pour fixer de manière sûre le raccord entre la paire de parties cadre (8256, 8256a, 8256b) de telle sorte que la paire de parties cadre (8256, 8256a, 8256b) tourne de manière coaxiale par rapport au raccord pendant que la paire de parties cadre (8256, 8256a, 8256b) se déplace entre la position déployée et la position rétrécie.

12. Dispositif implantable selon la revendication 11, la première pluralité de sutures passant plus d'une fois à travers l'œillet (8257) du raccord et l'œillet (8257) de chaque partie de la paire de parties cadre (8256, 8256a, 8256b).

13. Dispositif implantable selon la revendication 11 ou la revendication 12, comprenant en outre un manchon (8270) disposé autour de la paire de parties cadre (8256, 8256a, 8256b).

14. Dispositif implantable selon la revendication 13, au moins certaines de la première pluralité de sutures (8269), de la seconde pluralité de sutures ou des première et seconde pluralités de sutures passant à travers le manchon (8270) d'au moins une partie parmi la paire de parties cadre (8256, 8256a, 8256b).

15. Dispositif implantable selon l'une quelconque des revendications 11 à 14, chaque suture de la première pluralité de sutures (8269) étant fixée de manière sûre à elle-même.
